(19) Europäisches Patentamt
European Patent Office
Office européen des brevets

(11) **EP 2 260 763 A1**

(12) **EUROPEAN PATENT APPLICATION**
published in accordance with Art. 153(4) EPC

(43) Date of publication:
**15.12.2010 Bulletin 2010/50**

(51) Int Cl.:
*A61B 5/11* (2006.01)   *G01C 22/00* (2006.01)
*G06M 3/00* (2006.01)

(21) Application number: **09726642.3**

(22) Date of filing: **16.02.2009**

(86) International application number:
**PCT/JP2009/052579**

(87) International publication number:
**WO 2009/122788 (08.10.2009 Gazette 2009/41)**

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(30) Priority: **31.03.2008 JP 2008090976**
**18.11.2008 JP 2008294755**

(71) Applicant: **Sharp Kabushiki Kaisha**
**Osaka-shi, Osaka 545-8522 (JP)**

(72) Inventors:
• **FUJITA, Hidaka**
**Osaka-shi, Osaka 545-8522 (JP)**
• **SUGAHARA, Mariko**
**Osaka-shi, Osaka 545-8522 (JP)**

(74) Representative: **Müller - Hoffmann & Partner Patentanwälte**
**Innere Wiener Strasse 17**
**81667 München (DE)**

(54) **BODY MOTION MEASURING DEVICE, MOBILE TELEPHONE, METHOD FOR CONTROLLING THE BODY MOTION MEASURING DEVICE, BODY MOTION MEASURING DEVICE CONTROL PROGRAM, AND COMPUTER-READABLE RECORDING MEDIUM HAVING THE PROGRAM RECORDED THEREIN**

(57)   A portable terminal (1) includes: an X-axis direction body movement sensor (11), a Y-axis direction body movement sensor (12), and a Z-axis direction body movement sensor (13) for detecting accelerations in a plurality of directions; a scalarizing section (103) for combining the accelerations detected by the X-axis direction body movement sensor (11), the Y-axis direction body movement sensor (12), and the Z-axis direction body movement sensor (13), so as to obtain a combined value, and then scalarizing the combined value; a peak value detecting section (105) for obtaining, at every given time, the combined value obtained by the scalarizing section (103), and detecting an upper peak value and a lower peak value of the combined value thus obtained; and a step counting section (106) for counting another step when a difference between the upper peak value and the lower peak value which have been detected by the peak value detecting section (105) exceeds a given value. This makes it possible to accurately measure body movements without being affected by an error in a sensor and/or an error due to a change in offset voltage.

F I G. 1

EP 2 260 763 A1

**Description**

Technical Field

The present invention relates to: a body movement measuring device, especially a body movement measuring device which is capable of accurately carrying out measurement even if an error is made in a body movement sensor and/or an offset voltage; a mobile phone; a method for controlling the body movement measuring device; a body movement measuring device control program; and a computer-readable recording medium in which the body movement measuring device control program is recorded.

Background Art

A recent health boom has discovered new merits in walking. Further, many documents and the like state that it is good for health to walk with the aim of taking ten thousand steps per day. In view of this, many people use pedometers which are worn on wearers' bodies or the like for counting steps taken by the wearers.
A pedometer is worn on a human body or the like so that steps are counted by detecting body movements during walking. Accordingly, a detection sensitivity to body movements changes depending on at least where and in which direction the pedometer is worn. This makes it impossible to accurately count steps.
In view of the circumstances, a device has been suggested for counting body movements in accordance with body movements detected in a plurality of directions.
For example, Patent Literature 1 describes a body movement counting device for counting, as one time, a time interval between when an output, which is a combination of differences between (i) values of body movements detected in different directions and (ii) offset voltages, reaches not less than a predetermined given threshold and when the output reaches not more than the predetermined given threshold.

Patent Literature 1
Japanese Patent Application Publication, Tokukai, No. 2006-122573 A (Publication Date: May 18, 2006)
Patent Literature 2
Japanese Patent Application Publication, Tokukai, No. 2001-143048 A (Publication Date: May 25, 2001)

Summary of Invention

However, the aforementioned conventional arrangement causes the following problem. Namely, since each of sensors for detecting body movements in different directions has a possibility of error so that a larger or smaller value than a value that is supposed to be outputted may be outputted from the sensors. In addition, since an offset voltage corresponding to each of the sensors has a power supply voltage dependence and a temperature dependence, the offset voltage may change depending on a change in power supply voltage and temperature. Due to these reasons, a combined output which is a combination of outputs of the respective sensors for detecting body movements in different directions remains to be affected by an error in a sensor and/or an error due to a change in offset voltage (already described). Then, a case which should be counted as one step may not be counted as one step. On the contrary, a case which should not be counted as one step may be counted as one step.
The present invention has been made in view of the problems, and an object of the present invention is to realize a body movement measuring device and the like which is capable of accurately measuring body movements without being affected by an error in a sensor and/or an error due to a change in offset voltage.
In order to attain the object, a body movement measuring device in accordance with the present invention includes: an acceleration detecting section for detecting accelerations in a plurality of directions; scalarizing means for combining the accelerations detected by the acceleration detecting section, so as to obtain a combined value, and then scalarizing the combined value; peak value detecting means for obtaining, at every given time, the combined value obtained by the scalarizing means, and detecting an upper peak value and a lower peak value of the combined value thus obtained; and step counting means for counting another step when a difference between the upper peak value and the lower peak value which have been detected by the peak value detecting means exceeds a given value.
A method in accordance with the present invention for controlling a body movement measuring device including an acceleration detecting section for detecting accelerations in a plurality of directions, the method includes the steps of: (a) combining the accelerations detected by the acceleration detecting section, so as to obtain a combined value, and then scalarizing the combined value thus obtained; (b) sampling, at every given time, the combined value obtained in the step (a), and detecting an upper peak value and a lower peak value of the combined value thus sampled; and (c) counting another step when a difference between the upper peak value and the lower peak value which have been detected in the step (b) exceeds a given value.

Note here that a given value is regarded as human walking when a difference between an upper peak value and a lower peak value exceeds the given value.

According to the arrangement and the method, accelerations are detected in a plurality of directions. Then, the accelerations thus detected are combined and then scalarized. Subsequently, the combined value scalarized as a result of the combination is obtained at every given time, thereby detecting an upper peak value and a lower peak value of the combined value. Then, a case in which a difference between the upper peak value and the lower peak value which have been detected exceeds a given value is counted as one step.

According to this, it is possible to accurately carry out step counting even if (i) an error in a sensor for detecting an acceleration causes the sensor to output a larger or smaller value than a value which is supposed to be outputted and/or (ii) a change in offset voltage corresponding to each sensor causes a value corrected by the offset voltage to be larger or smaller than a value which is supposed to be set.

This is because, even if an error is made in a sensor for detecting an acceleration and/or an error is made due to a change in offset voltage corresponding to each sensor, a difference between an upper peak value and a lower peak value is unchanged since a combined value becomes larger or smaller as a whole in accordance with such an error.

For a fuller understanding of the nature and advantages of the invention, reference should be made to the ensuing detailed description taken in conjunction with the accompanying drawings.

Brief Description of Drawings

Fig. 1

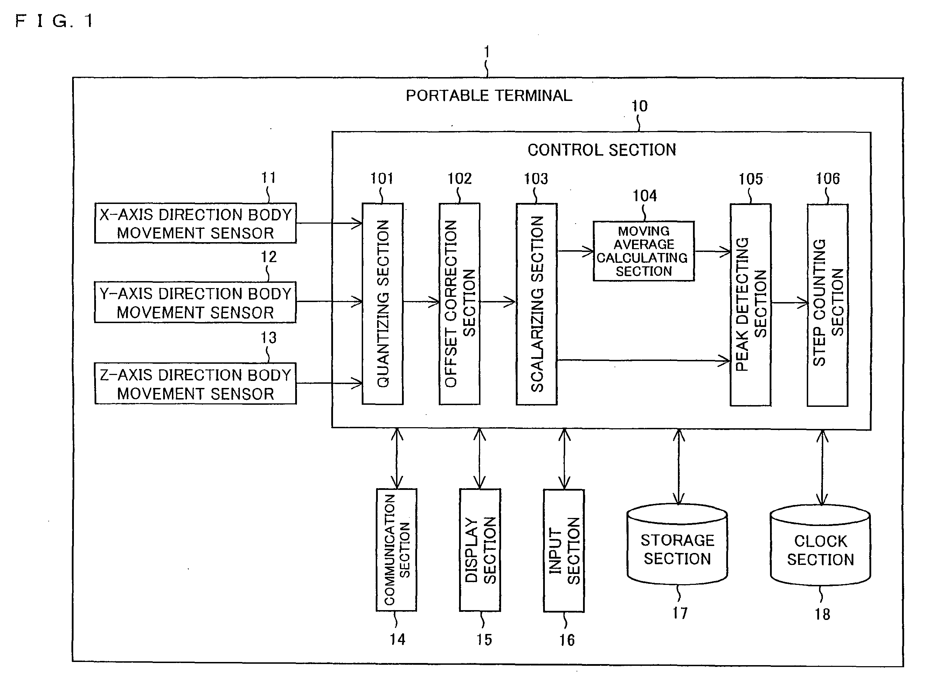

Fig. 1, which shows an embodiment of the present invention, is a block diagram illustrating an arrangement of a relevant part of a portable terminal.
Fig. 2

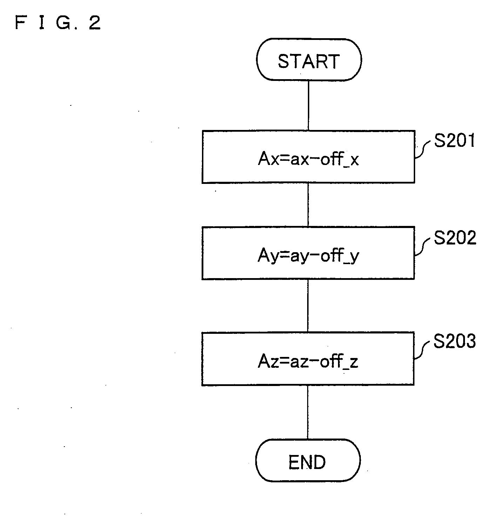

Fig. 2 is a flowchart illustrating a flow of a process of the embodiment which process is carried out by an offset correction section.
Fig. 3

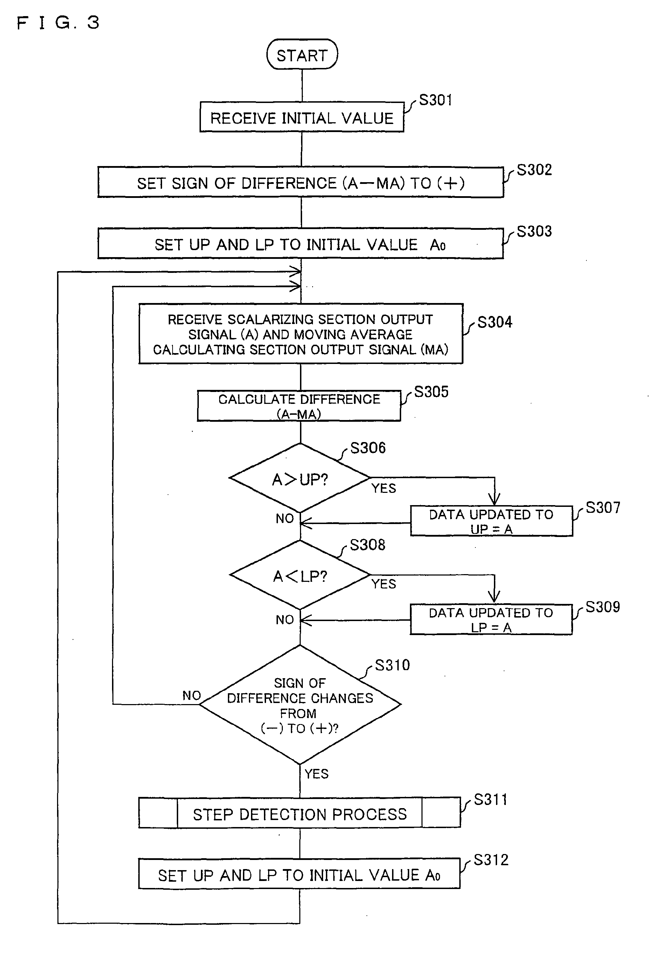

Fig. 3 is a flowchart illustrating a flow of a process of the embodiment in which process peak values (an upper peak value and a lower peak value) of an acceleration A is detected.
Fig. 4

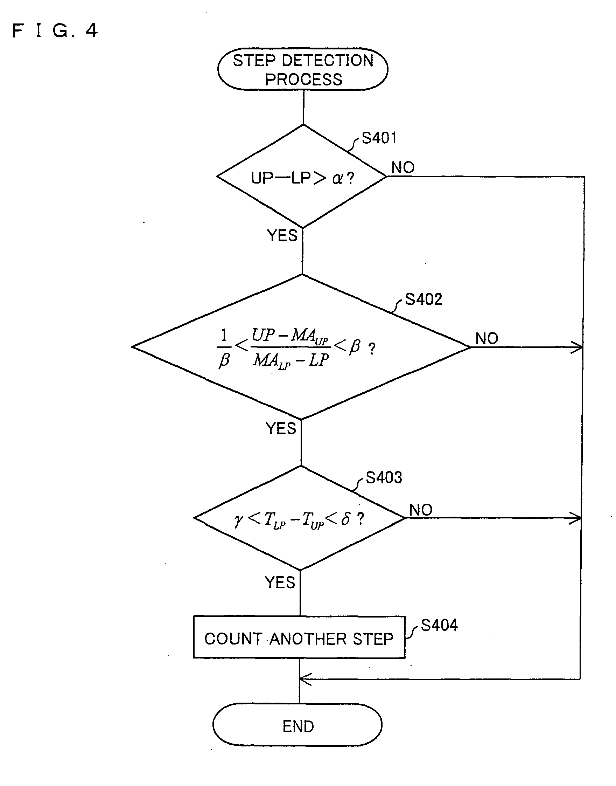

Fig. 4 is a flowchart illustrating a flow of a process of the embodiment in which process whether or not to count another step is determined.
Fig. 5

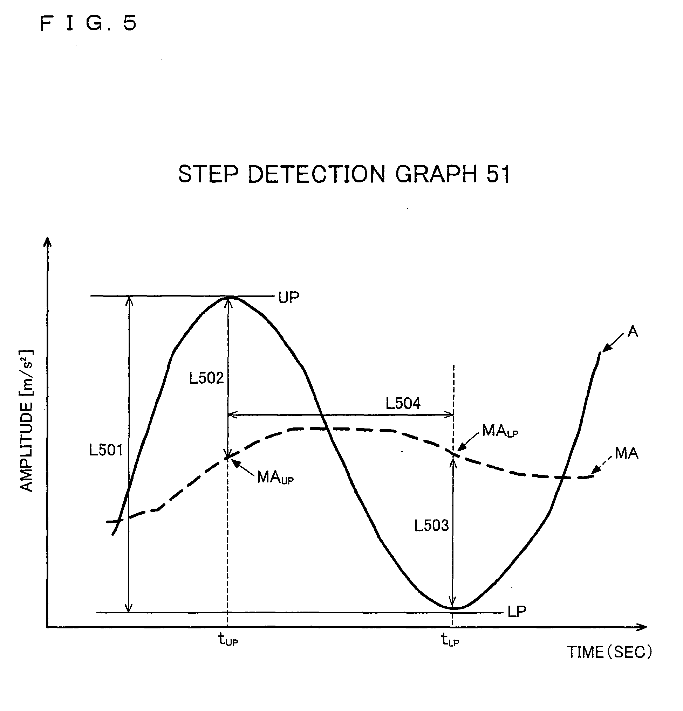

Fig. 5 is a graph illustrating a step detection process of the embodiment.
Fig. 6

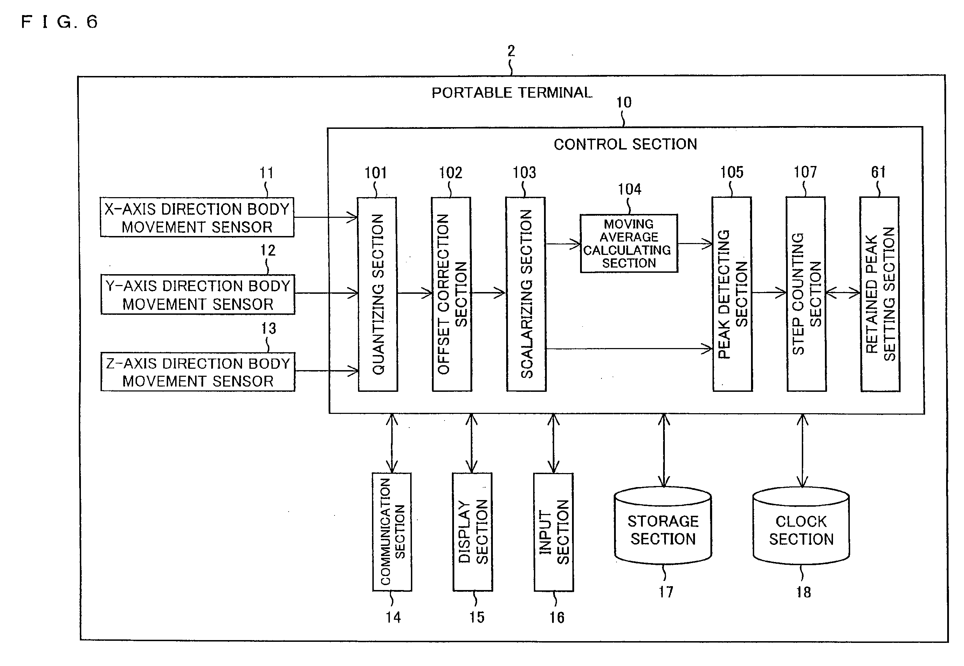

Fig. 6, which shows another embodiment of the present invention, is a block diagram illustrating an arrangement of a relevant part of a portable terminal.
Fig. 7

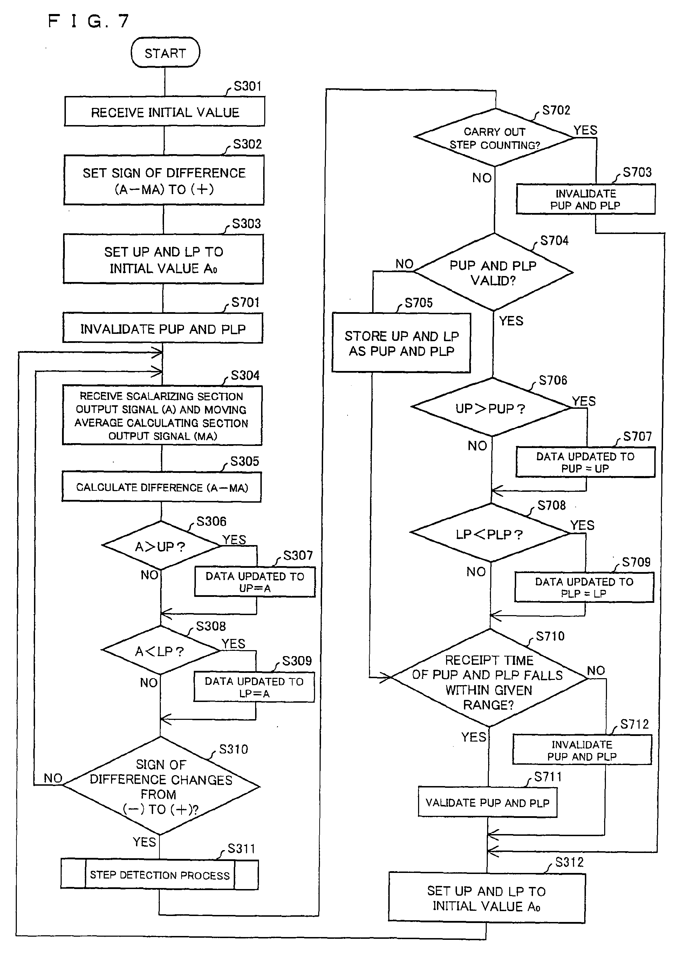

Fig. 7 is a flowchart illustrating a process of the another embodiment in which process a retained peak is set.
Fig. 8

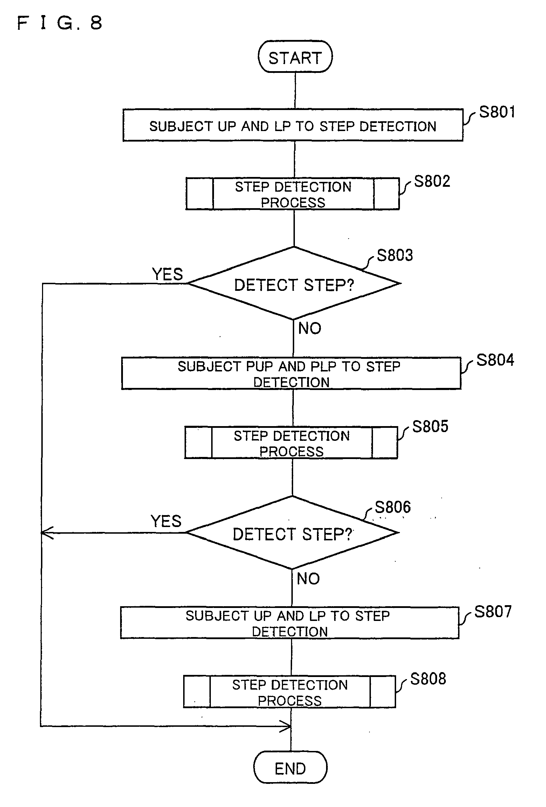

Fig. 8 is a flowchart illustrating a flow of a process of the another embodiment in which process an upper peak value and a lower peak value are selected for use in determination by a step counting section of whether or not to count another step.
Fig. 9

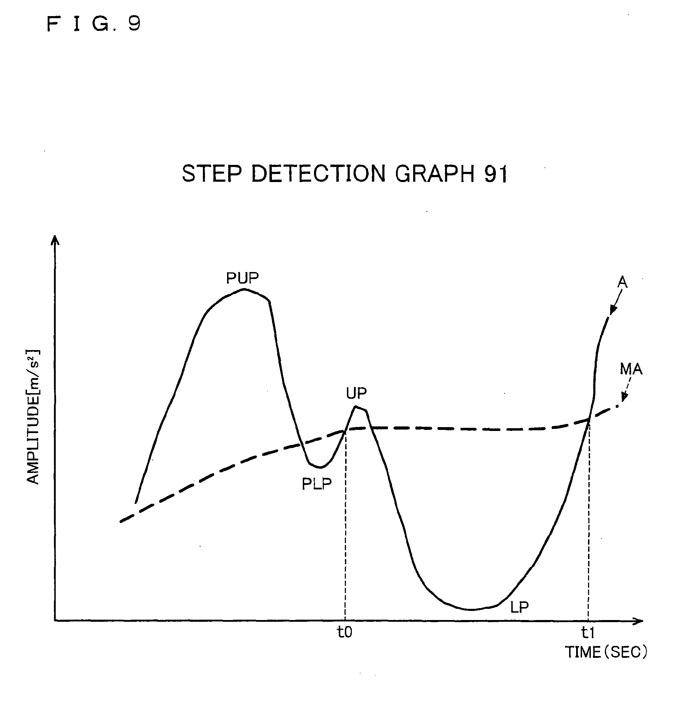

Fig. 9 is a graph illustrating an effect of the processes of the another embodiment.
Fig. 10

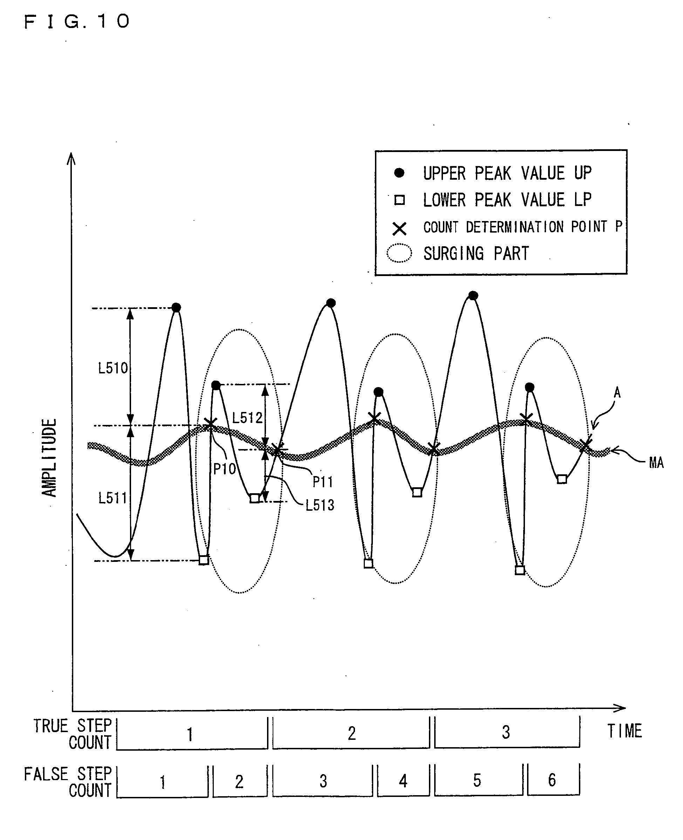

Fig. 10 is a graph illustrating a case of still another embodiment of the present invention in which case a surge meets a requirement for counting of another step.
Fig. 11

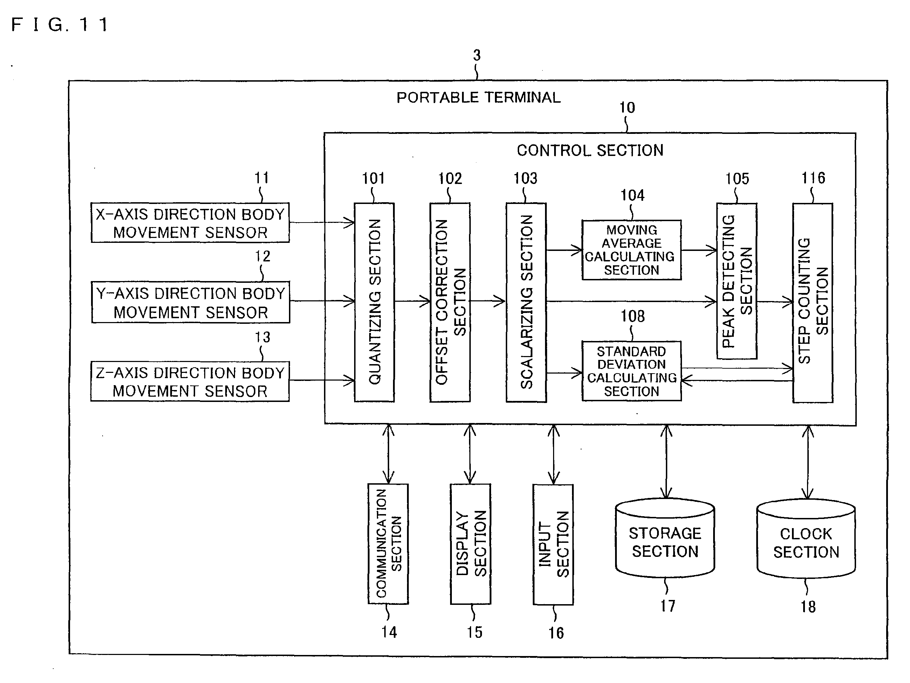

Fig. 11, which shows the still another embodiment, is a block diagram illustrating an arrangement of a relevant part of a portable terminal.
Fig. 12

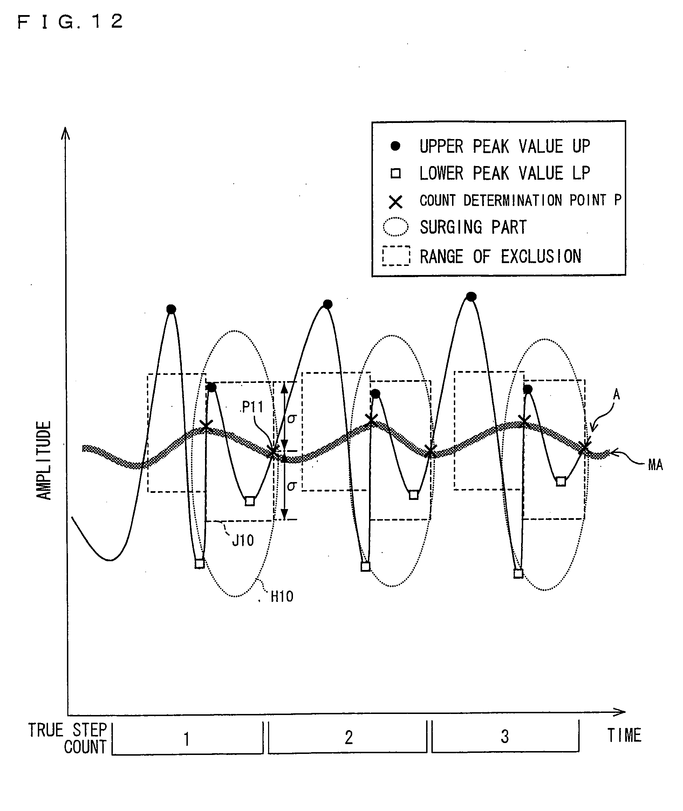

Fig. 12 is a graph illustrating a case of the still another embodiment in which case whether or not to carry out step counting is determined by use of a standard deviation.
Fig. 13

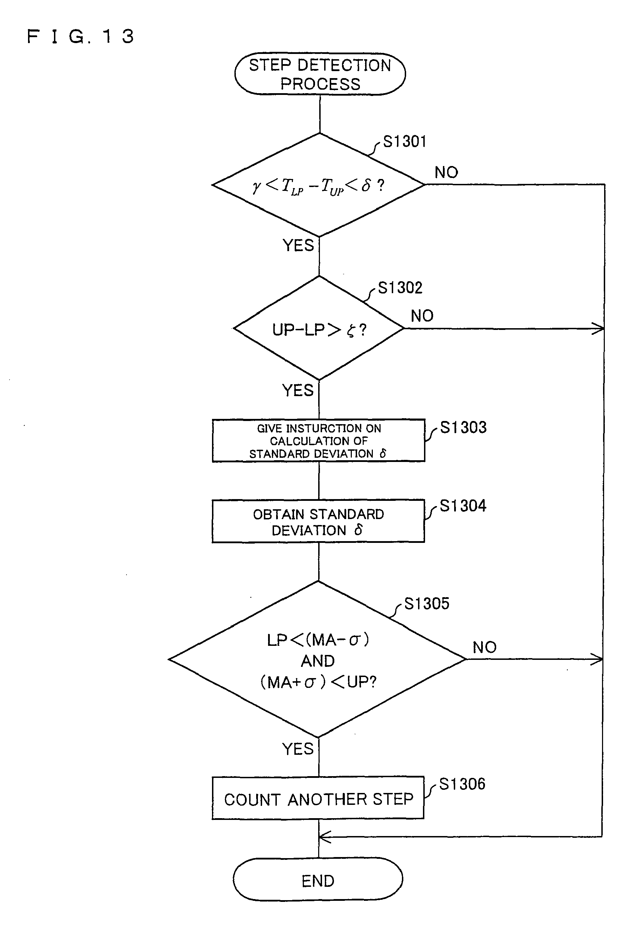

Fig. 13 is a flowchart illustrating a flow of a process of the still another embodiment in which process a step counting section determines whether or not to carry out step counting.

Fig. 14

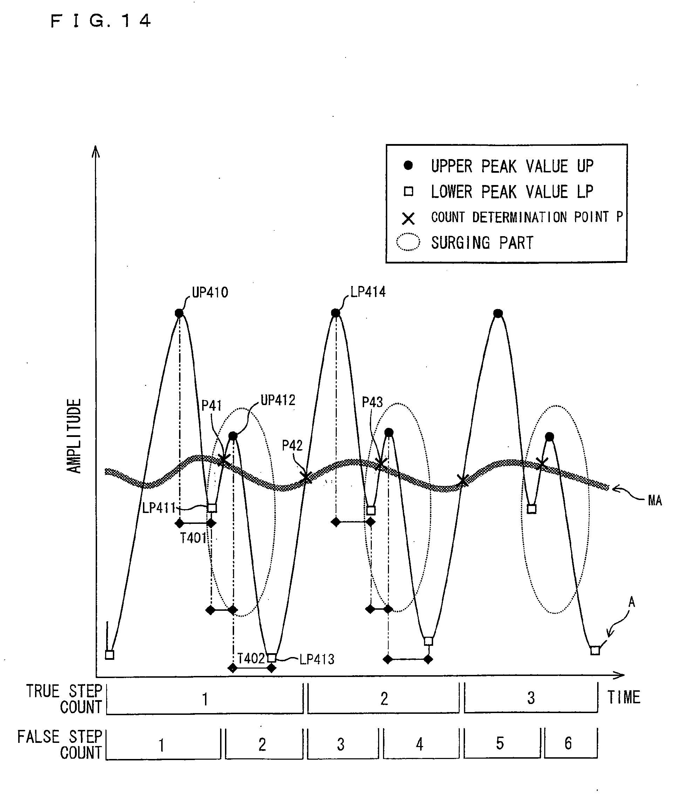

Fig. 14 is a graph illustrating a case of a further embodiment of the present invention in which case a surging part is counted as a step.

Fig. 15

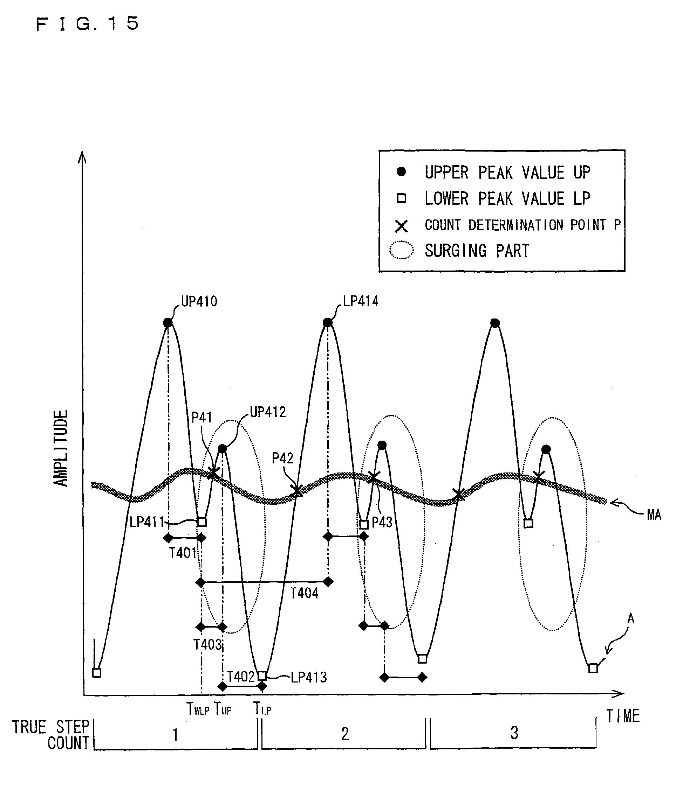

Fig. 15 is a graph illustrating a case of the further embodiment in which case whether or not to carry out step counting is determined by use of a requirement under which a period between (i) time of a lower peak value used for the last determination of step counting and (ii) time of an upper peak value detected this time is not less than a threshold.

Fig. 16

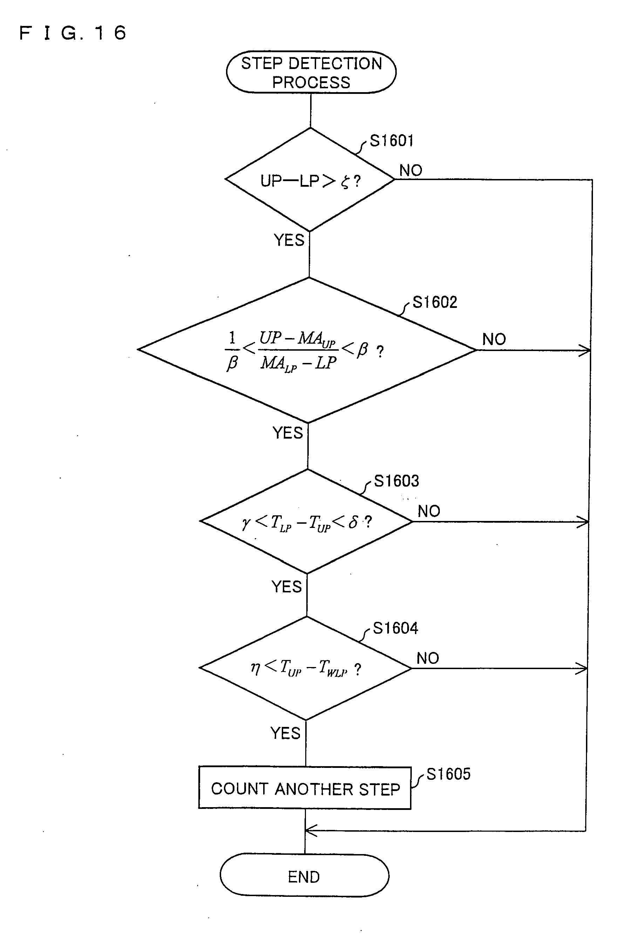

Fig. 16 is a flowchart illustrating a flow of a process of the further embodiment in which process a step counting section determines whether or not to carry out step counting.

Fig. 17

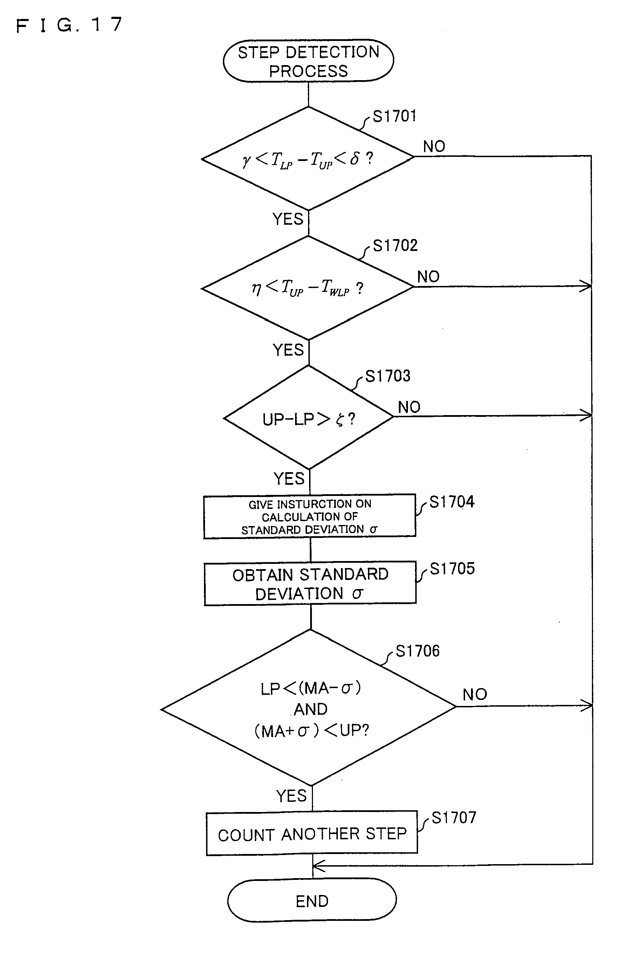

Fig. 17 is a flowchart illustrating a flow of a process of the further embodiment in which process a step counting section determines whether or not to carry out step counting.

Fig. 18

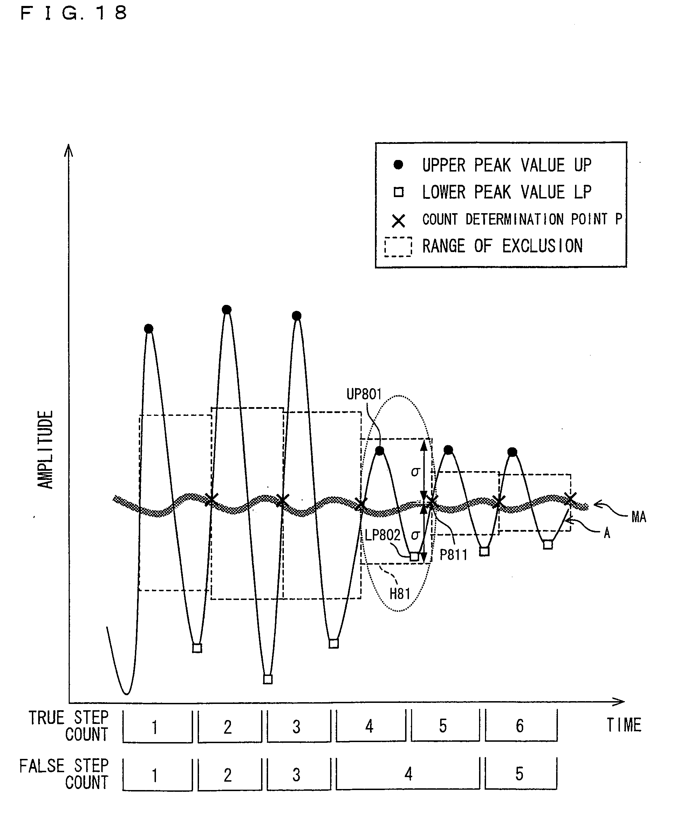

Fig. 18 is a graph illustrating a case of a still further embodiment of the present invention in which case another step that should be counted is not counted when a range of exclusion is defined by use of a standard deviation of an acceleration.

Fig. 19

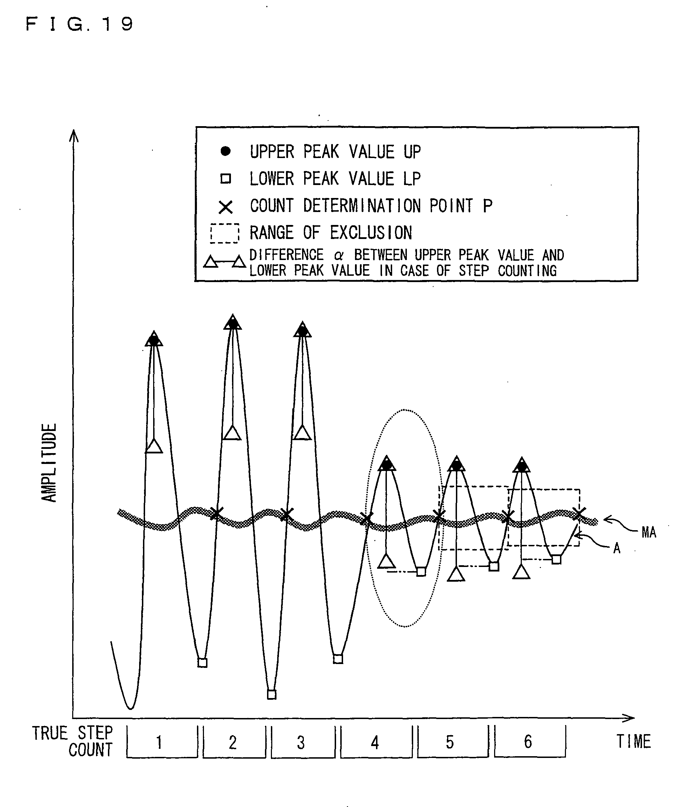

Fig. 19 is a graph illustrating a case of the still further embodiment in which case a step counting section carries out step counting.

Fig. 20

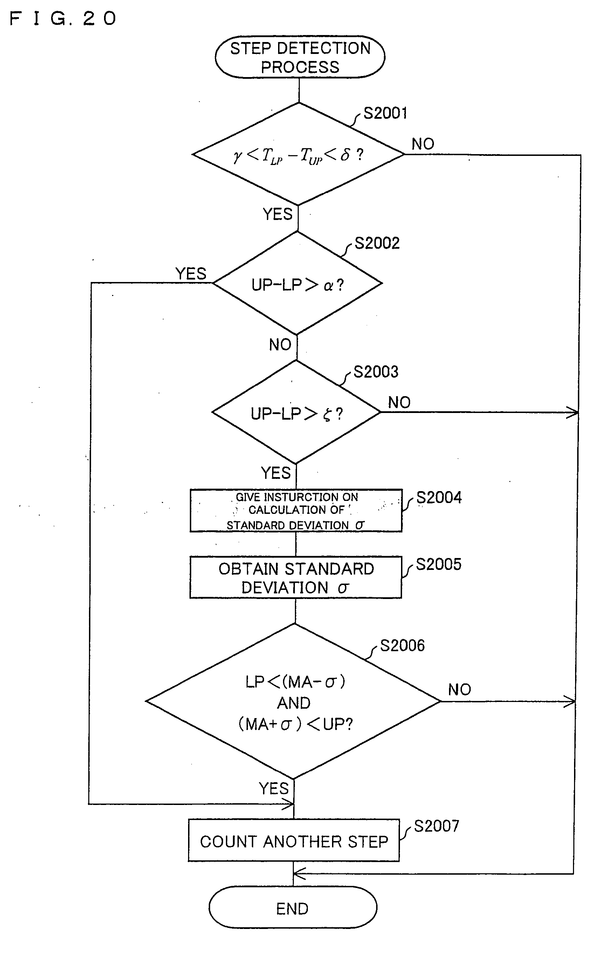

Fig. 20 is a flowchart illustrating a flow of a process of the still further embodiment in which process the step counting section determines whether or not to carry out step counting.

Fig. 21

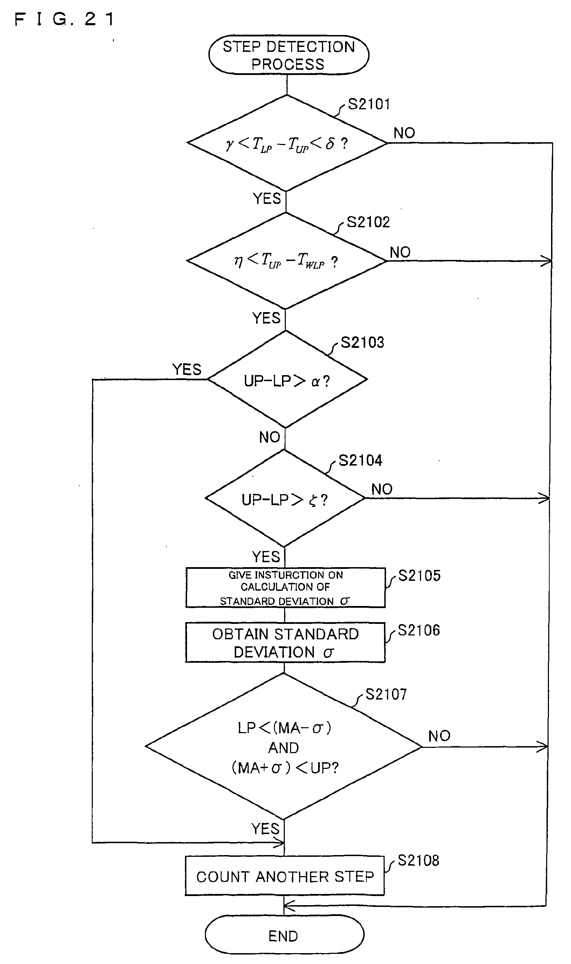

Fig. 21 is a flowchart illustrating a flow of a process of the still further embodiment in which process the step counting section determines whether or not to carry out step counting.

Reference Signs List

1, 2 Portable terminal (Body movement measuring device, Mobile phone)
10 Control section
11 X-axis direction body movement sensor (Acceleration detecting section)
12 Y-axis direction body movement sensor (Acceleration detecting section)
13 Z-axis direction body movement sensor (Acceleration detecting section)
101 Quantizing section (Quantizing means)
102 Offset correction section
103 Scalarizing section (Scalarizing means)
104 Moving average calculating section (Average calculating means)
105 Peak detecting section (Peak value detecting means)
106, 107, 116 Step counting section (Step counting means)
108 Standard deviation calculating section (Standard deviation calculating means)

Description of Embodiments

The following description discusses in more detail the present invention with reference to Examples and Comparative Examples. Note, however, that the present invention is not limited to these.

[First Embodiment]

An embodiment of the present invention is described below with reference to Figs. 1 through 5. Fig. 1 is a block diagram of a portable terminal (a body movement measuring device or a mobile phone) 1 in accordance with the present embodiment. The portable terminal 1 includes an X-axis direction body movement sensor (an acceleration detecting section) 11, a Y-axis direction body movement sensor (an acceleration detecting section) 12, a Z-axis direction body movement sensor (an acceleration detecting section) 13, a control section 10, a communication section 14, a display section 15, an input section 16, and a storage section 17 (see Fig. 1). The control section 10 includes a quantizing section (quantizing means) 101, an offset correction section 102, a scalarizing section (scalarizing means) 103, a moving average calculating section (average calculating means) 104, a peak detecting section (peak value detecting means) 105, and a step counting section (step counting means) 106.

According to the arrangement, the portable terminal 1 counts another step when a difference between an upper peak value and a lower peak value of a combined output value of accelerations detected in a given period exceeds a threshold. This can prevent a case which should be counted as one step but is not counted since a combined output value, which is affected by an error in each sensor and/or an error due to a change in offset voltage, does not exceed a given threshold.

Note that the portable terminal 1 also has a function as a mobile phone. This function, which is carried out by use of a publicly-known technique, is not to be described.

Next, each of the blocks of the portable terminal 1 is described below. Each of the X-axis direction body movement sensor 11, the Y-axis direction body movement sensor 12, and the Z-axis direction body movement sensor 13 is a body movement sensor for detecting an acceleration in a specific axial direction, thereby outputting a voltage caused due to the detection. The X-axis direction body movement sensor 11, the Y-axis direction body movement sensor 12, and the Z-axis direction body movement sensor 13 supply, to the quantizing section 101, their respective output voltages generated by acceleration detection. In the present embodiment, the X-axis direction body movement sensor 11, the Y-axis direction body movement sensor 12, and the Z-axis direction body movement sensor 13 are provided in the portable terminal 1 so that axes in their respective sensitivity directions cross each other orthogonally.

Note that it is unnecessary to separately provide, by axis, the X-axis direction body movement sensor 11, the Y-axis direction body movement sensor 12, and the Z-axis direction body movement sensor 13. It is only necessary to detect accelerations in directions of three axes which cross each other orthogonally. Therefore, it is possible to replace the X-axis direction body movement sensor 11, the Y-axis direction body movement sensor 12, and the Z-axis direction body movement sensor 13 with a sensor in which triaxial accelerations can be simultaneously detected in one housing.

The quantizing section 101 quantizes voltages supplied from the X-axis direction body movement sensor 11, the Y-axis direction body movement sensor 12, and the Z-axis direction body movement sensor 13 so that the voltages are digitalized. Then, quantized data (quantized voltages) is (are) transmitted to the offset correction section 102.

The quantizing section 101 is exemplified by an AD converter. For example, when an AD converter which has a quantization bit rate of 12 is used, voltages (analog values) supplied from the X-axis direction body movement sensor 11, the Y-axis direction body movement sensor 12, and the Z-axis direction body movement sensor 13 are converted to values (digital values) which are proportionate to voltages falling within a range of 0 to 4095. Note that these converted values are referred to as quantized voltages. Note also that it is desirable to quantize triaxial accelerations as simultaneously as possible.

The offset correction section 102 carries out an offset correction with respect to the quantized voltages received from the quantizing section 101 so that 0 (zero) is obtained when no accelerations exist in respective axial directions. Specifically, offset voltages preset for respective axes are subtracted from the quantized voltages received from the quantizing section 101. Then, the offset correction section 102 supplies, to the scalarizing section 103, the subtraction of the offset voltages from the quantized voltages received from the quantizing section 101. Accordingly, outputs from the offset correction section 102 correspond to accelerations in three directions of the portable terminal 1 to which accelerations gravitational accelerations are added.

Note here that offset voltages refer to quantized voltages obtained when the X-axis direction body movement sensor 11, the Y-axis direction body movement sensor 12, and the Z-axis direction body movement sensor 13 are respectively provided in an environment of 0G. For such an offset voltage, it is possible to use, for example, a quantized voltage observed during a free fall or a middle point of a maximum value and a minimum value of a quantized voltage observed when the portable terminal 1 is caused to rotate around an axis which is perpendicular to each of the axes.

A flow of a process carried out by the offset correction section 102 is described below with reference to Fig. 2. Fig. 2 is a flowchart illustrating the flow of the process carried out by the offset correction section 102. Note here that ax, ay, and az denotes quantized voltages in the X-axis, Y-axis, and Z-axis directions quantized by the quantizing section 101 and off_x, off_y, and off_z denotes preset offset voltages in the X-axis, Y-axis, and Z-axis directions. Note also that Ax, Ay, and Az are outputs from the offset correction section 102.

The offset correction section 102 finds and outputs an X-axis output Ax based on the equation of Ax = ax - off_x (S201), finds and outputs a Y-axis output Ay based on the equation of Ay = ay - off_y (S202), and finds and outputs a Z-axis output Az based on the equation of Az = az - off_z (S203). As described above, the offset correction section 102 outputs the X-axis output Ax, the Y-axis output Ay, and the Z-axis output Az.

The scalarizing section 103 converts, to one-dimensional scalars, the X-axis output Ax, the Y-axis output Ay, and the Z-axis output Az which have been supplied from the offset correction section 102. Then, the scalarizing section 103 supplies the scalar values thus converted to the moving average calculating section 104 and the peak detecting section 105.

In the present embodiment, scalarization is carried out by finding a square root of a sum of squares. Specifically, a scalarized acceleration A is found based on the following equation.

## [Math. 1]

$$A = \sqrt{A_x{}^2 + A_y{}^2 + A_z{}^2}$$

For the acceleration A supplied from the scalarizing section 103, the moving average calculating section 104 calculates a moving average MA obtained during a given period based on the following equation. Then the moving average calculating section 104 supplies the moving average MA thus calculated to the peak detecting section 105.

## [Math. 2]

$$MA(t) = \frac{1}{N} \sum_{i=0}^{N-1} A(t-i)$$

Note here that t denotes current time, N denotes the number of samples for calculating the moving average, A(t) denotes an acceleration supplied from the scalarizing section 103 at the current time t, and A (t - i) is an acceleration supplied from the scalarizing section 103 at time (t - i).

An interval for calculating the moving average MA is set as long as possible so that a vibration generated during walking due to a reason different from walking is less influential to an acceleration, but as short as possible so that a change in direction of an acceleration due to, for example, a change in direction of the portable terminal 1 can be followed. For example, in a case where the quantizing section 101 has a sampling frequency of 50Hz, the number of samples which corresponds to the interval for calculating the moving average can be set to 32.

This is drawn out by the following description. Assuming that people normally walk at a speed of 100 steps per minute, 600ms per step is obtained. In order that a center of a vibration generated in this cycle is followed, since 50Hz is equivalent to 20ms, the number of samples can be set to 32 based on the equation of 600/20 = 30 ($\approx$ 32). Note that this is merely a rough standard which need not be strictly met.

The peak detecting section 105 detects peak values (an upper peak value and a lower peak value) of the acceleration A supplied from the scalarizing section 103. Then, the peak detecting section 105 transmits, to the step counting section 106, the peak values together with time when the peak values are detected and values of MA which are obtained when the peak values are detected. Further, the peak detecting section 105 finds a difference between (i) the acceleration A supplied from the scalarizing section 103 and (ii) the moving average MA supplied from the moving average calculating section 104, so as to supply, to the step counting section 106, the difference as a differential signal.

The step counting section 106 carries out step counting in accordance with the peak values (the upper peak value and the lower peak value) received from the peak detecting section 105. In the present embodiment, when the differential signal supplied from the peak detecting section 105 changes a sign thereof from a negative sign to a positive sign, the step counting section 106 determines whether or not to carry out step counting. Note that a flow of a process is to be described later in which whether or not to carry out step counting is determined.

Every time the scalarizing section 103 and the moving average calculating section 104 supply the acceleration A and the moving average MA, respectively, the storage section 17 stores (i) the acceleration A supplied from the scalarizing section 103, (ii) the moving average MA supplied from the moving average calculating section 104, and (iii) time when (i) and (ii) are obtained. Further, the storage section 17 stores at least various data for use in the portable terminal 1 and various programs for causing the portable terminal 1 to operate.

The communication section 14 causes communication functions of the portable terminal 1 to be carried out. The display section 15 displays various states, an operation status, and a step detection result, and the like of the portable terminal 1.

The input section 16 is a user interface for receiving an instruction from a user to the portable terminal 1.

A clock section 18 has a function as a clock so that time is recognized in each of the blocks of the portable terminal 1.

Next, a flow of a process in which the peak values (the upper peak value and the lower peak value) of the acceleration A are detected is described below with reference to Fig. 3. Fig. 3 is a flowchart illustrating the process in which the peak values (the upper peak value and the lower peak value) of the acceleration A are detected.

First, the peak detecting section 105 receives (i) an initial value $A_0$ of the acceleration A from the scalarizing section 103 and (ii) an initial value $MA_0$ of the moving average MA from the moving average calculating section 104 (S301). Next, the peak detecting section 105 sets, to (+), an initial value of a sign of a difference between the acceleration A and the moving average MA (S302), and initial values of an upper peak value UP and a lower peak value LP of the acceleration A are respectively set to $A_0$ (S303).

Subsequently, the peak detecting section 105 receives subsequent sample values of the acceleration A and the moving average MA (S304). Then, the peak detecting section 105 finds the difference (A - MA) (S305), and determines whether or not the acceleration A received is larger than the upper peak value UP (A > UP) (S306). In a case where A > UP (YES at S306), the peak detecting section 105 regards the acceleration A as the upper peak value UP, thereby updating data on the acceleration A and receipt time of the acceleration A (S307). Thereafter, the process proceeds to S308.

In contrast, in a case where A ≤ UP (NO at S306), the peak detecting section 105 determines whether or not the acceleration A received is smaller than the lower peak value LP (A < LP) (S308). In a case where A < LP (YES at S308), the peak detecting section 105 regards the acceleration A as the lower peak value LP, thereby updating data on the acceleration A and the receipt time of the acceleration A (S309). Thereafter, the process proceeds to S310.

In contrast, in a case where A ≥ LP (NO at S308), the peak detecting section 105 determines whether or not the sign of the difference (A - MA) changes from (-) (last time) to (+) (this time) (S310). In a case where the sign of the difference (A - MA) changes from (-) to (+) (YES at S310), a step detection process is carried out (S311). In contrast, the sign of the difference (A - MA) does not change from (-) (last time) to (+) (this time) (NO at S310), the process returns to S304, so that the peak detecting section 105 receives subsequent sample values.

When the step detection process is ended, the upper peak value UP and the lower peak value LP are respectively set to the initial values $A_0$ (S312). Thereafter, the process returns to S304, so that the peak detecting section 105 receives subsequent sample values.

Next, a flow of a process in which whether or not to count another step is determined is described below with reference to Fig. 4. Fig. 4 is a flowchart illustrating the flow of the process in which whether or not to count another step is determined.

First, the step counting section 106 determines whether or not a difference between an upper peak value UP and a lower peak value LP which have been received is larger than a threshold a (UP - LP > $\alpha$) (S401). In a case where UP - LP > a (YES at S401), the step counting section 106 subsequently determines whether or not a ratio of a difference between the upper peak value UP and a corresponding moving average $MA_{UP}$ to a difference between the lower peak value LP and a corresponding moving average $MA_{UP}$ falls within a threshold range of $1/\beta < \{(UP - MA_{UP})/(MA_{LP} - LP)\} < \beta$ (S402). In a case where $1/\beta < \{(UP - MA_{UP})/(MA_{LP} - LP)\} < \beta$ (YES at S402), the step counting section 106 determines whether or not a period between the upper peak value UP and the lower peak value LP ($T_{LP} - T_{UP}$) falls within a given range of $\gamma < T_{LP} - T_{UP} < \delta$ (S403). In a case where $\gamma < T_{LP} - T_{UP} < \delta$ (YES at S403), the step counting section 106 determines that the change is one step, thereby counting another step (S404). Thereafter, the process proceeds to S312.

The aforementioned step counting process is described below with reference to a step detection graph 51 in which a vertical axis indicates an acceleration and a horizontal axis indicates time. Fig. 5 illustrates the step counting process.

In the step detection graph 51, A denotes an acceleration scalarized by the scalarizaing section 103 and MA denotes a moving average calculated by the moving average calculating section 104. Further, $MA_{UP}$ denotes a value of the moving average MA which value is obtained when the acceleration A has the upper peak value UP and $MA_{LP}$ denotes a value of the moving average MA which value is obtained when the acceleration A has the lower peak value LP.

First, at S401 in Fig. 4, it is determined (i) whether or not a difference L501 between UP and LP is larger than the threshold a. Next, at S402 in Fig. 4, it is determined (ii) whether or not the ratio of a difference L502 between the upper peak value UP and the corresponding moving average $MA_{UP}$ to a difference L503 between the lower peak value LP and the corresponding moving average $MA_{LP}$ falls within a given range of $1/\beta < (L502)/(L503) < \beta$. Finally, at S403 in Fig. 4, (iii) whether or not a period L504 between the upper peak value UP and the lower peak value LP ($T_{LP} - T_{UP}$) falls within a given range of $\gamma < L504 < \delta$. Then, when these three requirements (i) through (iii) are met, another step is counted.

Note that an actually set value of the threshold a changes depending on a sensitivity of an acceleration sensor and a quantization bit rate of an AD converter. For example, in a case where the acceleration sensor has a sensitivity which is a tenth of a power supply voltage per 1G and the AD converter has a quantization bit rate of 12, it is shown that the threshold a equivalent to 0.35G causes an increase in accuracy when a process for step counting is actually carried out. In view of this, in a case where it is set that $4096 \times (1/10) \times 0.35 = 143$, the threshold a is equivalent to 0.35G.

Similarly, the threshold $\beta$ can be set to "4", the threshold $\gamma$ can be set to "100ms", and the threshold $\delta$ can be set to "500ms".

As described earlier, according to the embodiment, in a case where a difference between an upper peak (the upper peak value) and a lower peak (the lower peak value) of the acceleration A which has been scalarized, i.e., an amplitude of a waveform formed by the acceleration A is larger than a threshold, the case is counted as one step. According to this, it is possible to prevent an influence on step counting even if, due to an error in a body movement sensor, an output has a larger value than a value which is supposed to be outputted or a smaller value than the value which is supposed to be outputted. In addition, even if an offset voltage is deviated from a value which is supposed to be set, it is similarly possible to prevent an influence on step counting.

In the step detection graph 51, "the ratio of the difference L502 between UP and $MA_{UP}$ to the difference L503 between LP and $MA_{LP}$ falls within a threshold range" is a requirement for counting of another step. According to this, in a case where an acceleration caused by some external factor or an acceleration which is not suitable for step counting is detected, it is possible to exclude a result of the detection from step counting.

Further, in the step detection graph 51, "the period L504 between UP and LP falls within the given range" is a requirement for counting of another step. According to this, it is possible to exclude a vibration which takes time during which human walking cannot be carried out.

In the present embodiment, a comparison between values of the acceleration A was carried out, so as to find UP and LP. How to find UP and LP is not limited to this. For example, an upper peak (or a lower peak) of (i) a difference between the acceleration A and the moving average MA or (ii) a difference between the acceleration A and a value found based on another method can be found as UP or LP.

In the present embodiment, "the difference between UP and LP, i.e., L501 is larger than the threshold $\alpha$" is a requirement for counting of another step. A requirement for counting of another step is not limited to this. It is only necessary that the waveform of the acceleration A have an amplitude which is at not less than a given level. For example, "both a difference between UP and an average and a difference between LP and the average are larger than a threshold" can also be a requirement for counting of another step.

In the present embodiment, "the ratio of the difference L502 between UP and $MA_{UP}$ to the difference L503 between LP and $MA_{LP}$ falls within the threshold range" is a requirement for counting of another step. A requirement for counting of another step is not limited to this. It is only necessary that an amplitude ratio from a substantial center to a peak of a vibration fall within a threshold range. It is also possible to replace the moving average calculating section 104 with an average calculating section (not illustrated) or a median calculating section (median calculating means, not illustrated) calculating section, thereby using, for example, another average found by the average calculating section or a median found by the median calculating section instead of the moving average MA. In many cases, use of a median as a representative value is more suitable for representing tendency as a whole, as compared to the case in which an average is used. Therefore, in a case where a peak value is extremely large or small due to some external factor or the like, it is possible to carry out step counting with a reduced influence of the peak value.

In the present embodiment, an example in which a moving average in which weighting by time is equally carried out is used as an average of the acceleration A is described. An average of the acceleration A is not limited to this. It is only necessary to find a value at a substantial center of a vibration. For example, it is possible to use a weighted moving average in which weighting is changed by time.

In the present embodiment, determination of walking is carried out at "a timing of a change in sign of the difference between A and MA (A - MA) from (-) to (+)". A timing at which determination of walking is carried out is not limited to this. It is only necessary to carry out determination of walking for every one cycle of the waveform formed by the acceleration A. For example, determination of walking can be carried out at a timing of a change in sign of the difference changes from (+) to (-) or a timing at which the acceleration A has an upper peak value or a lower peak value.

In the present embodiment, "the period between UP and LP, i.e., L504 falls within the given range" is a requirement for counting of another step. A requirement for counting of another step is not limited to this. It is only necessary to associate counting of another step with a specific cycle such as a half cycle or one cycle of walking. For example, it is only necessary that (i) a period between UP and subsequent UP or (ii) an interval in which the sign of the difference between the acceleration A and the moving average MA (A - MA) changes from (-) to (+) fall within a given range.

[Second Embodiment]

Another embodiment of the present invention is described below with reference to Figs. 6 through 9. Note that, for convenience, members having functions identical to those of the respective members illustrated in the First Embodiment are given respective identical reference numerals, and a description of those members is omitted here.

In the First Embodiment, the step counting section 106 carries out step counting in an interval as long as one cycle from a point of a change in sign of the difference between the acceleration A supplied from the scalarizing section 103 and the moving average MA supplied from the moving average calculating section (A - MA) from (-) to (+) to a point of a subsequent change in sign of the difference between the acceleration A and the moving average MA (A - MA) from (-) to (+).

However, during the interval as long as one cycle, an external factor or the like which is not related to walking may cause a change in acceleration A, thereby producing an upper peak value and a lower peak value of the acceleration A. This causes a change in sign of the difference between the acceleration A and the moving average MA from (-) to (+), so that determination of walking is carried out (S310 and S311 of Fig. 3). However, in this case, the three requirements described in the First Embodiment (S401, S402, and S403 of Fig. 4) are not met, so that step counting may not be carried out.

Namely, in the First Embodiment, when an upper peak value and a lower peak value are produced due to the aforementioned change in acceleration A so that the moving average MA exists between the upper peak value and the lower peak value, a step which is supposed to be counted may not be counted.

In view of the circumstances, in the present embodiment, in a case where the step counting section 106 does not count another step at S311 of Fig. 3, UP and LP which have been received are retained as retained peaks, so that subsequent determination of whether or not to count another step is carried out by use of the retained peaks.

The present embodiment is different from the First Embodiment in that the step counting section 106 of the First Embodiment is replaced with a retained peak setting section 61 and a step counting section (step counting means) 107 (see Fig. 6). Fig. 6 is a block diagram illustrating an arrangement of a relevant part of a portable terminal (a body movement measuring device) 2 according to the present embodiment.

In a case where the step counting section 107 does not carry out step counting, the retained peak setting section 61 retains, as a retained upper peak value PUP and a retained lower peak value PLP, UP and LP which were used for determination of whether or not to carry out step counting. The retained peak setting section 61 carries out setting of validation and invalidation of retained peaks. The step counting section 107 determines whether or not to count another step by use of UP and LP which have been received from a peak detecting section 105 and the retained peaks PUP and PLP. A process in which the step counting section 107 counts another step is to be described later.

Next, a flow of a process in which retained peaks are set is described below with reference to Fig. 7. Fig. 7 is a flowchart illustrating the flow of the process in which retained peaks are set. Note that steps identical to the steps illustrated in Fig. 3 are given respective identical reference numerals, and a description of those steps is omitted here.

First, S303 is followed by a step (S701) in which the retained peak setting section 61 invalidates the retained upper peak value PUP and the retained lower peak value PLP. Then, the process proceeds to S304.

S311 is followed by a step (S702) in which the retained peak setting section 61 determines whether or not step counting has been carried out by the step counting section 107. In a case where step counting has been carried out (YES at S702), the retained peak setting section 61 invalidates the retained upper peak value PUP and the retained lower peak value PLP (S703). Then, the process proceeds to S312.

In contrast, in a case where no step counting has been carried out (NO at S702), the retained peak setting section 61 determines whether or not the retained peaks are valid (S704). In a case where the retained peaks are invalid (NO at S704), the retained peak setting section 61 stores, as the retained upper peak value PUP and the retained lower peak value PLP, UP and LP which have been received. Then, the process proceeds to S710.

In contrast, in a case where the retained peaks are valid (YES at S704), the retained peak setting section 61 determines whether or not the upper peak value UP received is larger than the retained upper peak value PUP which is valid (UP > PUP) (S706). In a case where UP > PUP (YES at S706), data is updated so that the upper peak value UP received is the retained upper peak value PUP (S707). Then, the process proceeds to S708. In contrast, in a case where UP $\leq$ PUP (NO at S706), the process proceeds to S708.

Next, the retained peak setting section 61 determines whether or not the lower peak value LP received is smaller than the retained lower peak value PLP which is valid (LP < PLP) (S708). In a case where LP < PLP (YES at S708), data is updated so that the lower peak value LP received is the retained lower peak value PLP (S709). Then, the process proceeds to S710. In contrast, in a case where LP $\geq$ PLP (NO at S708), the process proceeds to S710.

Thereafter, the retained peak setting section 61 makes a comparison of receipt time and current time for each of the retained upper peak value PUP and the retained lower peak value PLP, thereby determining whether or not a period between the receipt time and the current time is not more than a threshold $\varepsilon$ (S710). In a case where the difference between the receipt time and the current time is not more than the threshold $\varepsilon$ (YES at S710), the retained peak setting section 61 validates the retained upper peak value PUP and the retained lower peak value PLP (S711). Then, the process proceeds to S312. In contrast, in a case where the difference between the receipt time and the current time is larger than the threshold $\varepsilon$ (NO at S710), the retained peak setting section 61 invalidates the retained upper peak value PUP and the retained lower peak value PLP (S712). Then, the process proceeds to S312.

The threshold $\varepsilon$ is set to, for example, 1000ms.

Next, the following description discusses, with reference to Fig. 8, a flow of a process in which an upper peak value and a lower peak value are selected for use in determination by the step counting section 107 of whether or not to count another step. Fig. 8 is a flowchart illustrating the flow of the process in which an upper peak value and a lower peak value are selected for use in determination by the step counting section 107 of whether or not to count another step. This process is carried out at S311 of the flowchart illustrated in Fig. 7.

Similarly to the step counting section 106, the step counting section 107 also makes a comparison of an upper peak value and a lower peak value, thereby determining whether or not to carry out step counting. Note, however, that the step counting section 107, differently from the step counting section 106, selects, from the upper peak value UP and the lower peak value LP which have been received, the retained upper peak value PUP, and the retained lower peak value PLP, an upper peak value and a lower peak value which are subjected to the determination.

First, the step counting section 107 sets the upper peak value and the lower peak value which are subjected to the determination of whether or not to carry out step counting to the upper peak value UP and the lower peak value LP which have been received from the peak detecting section 105 (S801). Then, the step detection process is carried out in accordance with the flow illustrated in Fig. 4 (S802). In a case where the step detection is carried out (YES at S803), the selection process is ended.

In contrast, in a case where no step detection is carried out (NO at S803), the step counting section 107 sets the upper peak value and the lower peak value which are subjected to the determination of whether or not to carry out step counting to the retained upper peak value PUP and the lower peak value LP which has been received from the peak detecting section 105 (S804). Then, the step detection process is carried out in accordance with the flow illustrated in Fig. 4 (S805). In a case where the step detection is carried out (YES at S806), the selection process is ended.

In contrast, in a case where no step detection is carried out (NO at S806), the step counting section 107 sets the upper peak value and the lower peak value which are subjected to the determination of whether or not to carry out step counting to the upper peak value UP which has been received from the peak detecting section 105 and the retained lower peak value PLP (S807). Then, the step detection process is carried out in accordance with the flow illustrated in Fig. 4 (S808). Thereafter, the selection process is ended.

An effect of the aforementioned processes is described below with reference to a step detection graph 91 in which a vertical axis indicates an acceleration and a horizontal axis indicates time. Fig. 9 is a graph illustrating the effect of the processes of the present embodiment.

Assume that an acceleration A scalarized by a scalarizing section 103 and a moving average MA supplied from a moving average calculating section 104 have values illustrated in the step detection graph 91. In the First Embodiment, first, at time to, the step detection process is carried out with respect to PUP and PLP which serve as the upper peak value and the lower peak value which are subjected to the step detection process. However, since the aforementioned requirements are not met, no step counting is carried out. Subsequently, at time t1, the step detection process is carried out with respect to UP and LP which serve as the upper peak value and the lower peak value which are subjected to the step detection process. However, since the aforementioned requirements are not met, either, no step counting is carried out.

On the other hand, in the present embodiment, no step counting is carried out as well at the time to, similarly to the case of the First Embodiment. In contrast, step counting is carried out at the time t1 since the step detection process is carried out with respect to PUP and LP which serve as the upper peak value and the lower peak value which are subjected to the step detection process.

Accordingly, even if an upper peak value and a lower peak value (e.g., PLP and UP which are illustrated in the step detection graph 91) are produced, during walking, by some influence that is not related to walking, it is possible to accurately count another step. This makes it possible to more accurately carry out step detection.

Note that, in the present embodiment, only a pair of a retained upper peak value and a retained lower peak value is stored for use in determination of step detection. A plurality of such pairs can be stored for use in the determination, thereby causing an increase in accuracy of step detection.

For example, up to 10 retained upper peak values and up to 10 retained lower peak values are storable. When the aforementioned requirements are met in the steps from (S706) through (S709), data on a retained upper peak value and a retained lower peak value is not updated but another retained upper peak value and another retained lower peak value are added as long as the number of respective retained upper peak values and retained lower peak values does not exceed 10. Then, the process of the flow illustrated in Fig. 4 is repeatedly carried out in the step detection process by combining, one by one, the retained upper peak values and the retained lower peak values thus accumulated.

In this case, when n retained upper peak values and m retained lower peak values are stored, $n \times m$ combinations are possible. Then, the aforementioned determination is carried out with respect to each of the combinations. In a case where step counting is carried out, the determination is finished.

Further, it is possible not only to store an upper peak value and a lower peak value as a retained upper peak value and a retained lower peak value as they are but also to store each of the retained upper peak value and the retained lower peak value as a weighed centroid for use in the determination. Such use refers to use of virtual centroids of a respective plurality of retained upper peak values and retained lower peak values as ones of upper peak values and lower peak values which ones are subjected to the requirements for step detection. It is possible to use such a method employing a virtual centroid in combination with a retained upper peak value and a retained lower peak value.

It is an object of the present embodiment to prevent a case in which, in the First Embodiment, the requirements for counting of another step are not met, thereby making it impossible to count another step that should be counted. An object of the present embodiment is not limited to this. The present embodiment is also applicable to removal of an influence of a change in waveform of the acceleration A which change is caused by an external factor or the like.

[Third Embodiment]

Still another embodiment of the present invention is described below with reference to Figs. 10 through 13. Note that, for convenience, members having functions identical to those of the respective members illustrated in the respective First and Second Embodiments are given respective identical reference numerals, and a description of those members is omitted here.

The present embodiment is directed to accurately carry out step counting in view of a surge in the graphic curve in association with but not caused by walking. Note here that in this description a surge in the graphic curve refers to a case in which, though the surge is not cause by walking, a relationship between an upper peak value and a lower peak value of a waveform of an acceleration A is similar to a relationship between an upper peak value and lower peak value which relationship should be counted as a step.

In each of the First and Second Embodiments, the step counting section 106 counts another step, provided that the ratio of the difference between the upper peak value UP and the corresponding moving average $MA_{UP}$ to the difference between the lower peak value LP and the corresponding moving average $MA_{LP}$ falls within the threshold range.

However, there also exists a surge in which the ratio of the difference between the upper peak value UP and the corresponding moving average $MA_{UP}$ to the difference between the lower peak value LP and the corresponding moving average $MA_{LP}$ falls within the threshold range. In this case, the surge, which is not caused by walking, meets the requirement for counting of another step. Accordingly, only the requirement under which the ratio of the difference between the upper peak value UP and the corresponding moving average $MA_{UP}$ to the difference between the lower peak value LP and the corresponding moving average $MA_{LP}$ falls within the threshold range is insufficient to prevent a surge from being counted as a step.

Note that, $MA_{UP}$ and $MA_{LP}$, which can be replaced with a moving average MA obtained at the time of determination of walking, are described below by use of the moving average MA.

A case in which a surge meets the requirement for counting of another step is described below with reference to Fig. 10. Fig. 10 is a graph illustrating the case in which a surge meets the requirement for counting of another step.

Fig. 10 illustrates the waveform of the acceleration A, on which waveform a black dot denotes an upper peak value UP, a square outline with a blank inside denotes a lower peak value LP, and a cross ($\times$) denotes a count determination point P. Further, in Fig. 10, a part surrounded by an ellipse is a surging part. Note that L510 shows a difference between an upper peak value UP and the moving average MA at a count determination point P10 and L511 shows a difference between a lower peak value LP and the moving average MA at the count determination point P10.

Note also that L512 shows a difference between an upper peak value UP and the moving average MA at a count determination point P11 and L513 shows a difference between a lower peak value LP and the moving average MA at the count determination point P11.

In a case where a surging part is similar in shape to a part which is counted as a step, a ratio of L512 to L513 is substantially identical to a ratio of L510 to L511 (see Fig. 10). Accordingly, a surging part H10 is counted as a step when the other requirements for counting of another step are met. This makes it impossible to accurately carry out step counting. For example, in the case of the waveform illustrated in Fig. 10, steps may be falsely counted as 6 though the steps are actually 3 in total.

In view of the circumstances, in the present embodiment, a range in which there exist no upper peak value and no lower peak value for determination of whether or not to carry out step counting is defined, thereby preventing the aforementioned surge from meeting the requirements for counting of another step. An upper peak value and a lower peak value of a surging part are smaller than an upper peak value and a lower peak value of a part which is counted as a step. Accordingly, a range in which an upper peak value and a lower peak value are smaller than the upper peak value and the lower peak value of the part which is counted as a step and larger than the upper peak value and the lower peak value of the surging part is defined as the range in which there exist no upper peak value and no lower peak value for determination of whether or not to carry out step counting.

This point is specifically described with reference to Fig. 11. Fig. 11 is a block diagram illustrating a portable terminal 3 according to the present embodiment. The portable terminal 3 is different in arrangement from the portable terminal 1 in that a control section 10 includes a standard deviation calculating section 108 and the step counting section 106 is replaced with a step counting section 116.

The standard deviation calculating section 108 causes a storage section 17 to store a value of the acceleration A which value is obtained from a scalarizing section 103. In response to an instruction from the step counting section 116 to calculate a standard deviation σ, the standard deviation calculating section 108 calculates the standard deviation σ of values of the acceleration A which values are obtained during a set time interval stored in the storage section 17. In the present embodiment, in which a sampling cycle is 30ms, the standard deviation σ of the latest 32 values of the acceleration A are calculated. Thereafter, the standard deviation σ thus calculated is transmitted to the step counting section 116.

A relationship between the sampling cycle and the number of values (the number of samples) of the acceleration A which are necessary to accurately carry out step counting is described here. For example, assume that the upper limit of the sampling cycle to which the number of samples correspond is 80 steps/min. Samples for at least one step are necessary to calculate the standard deviation σ, similarly to the case in which the moving average MA is calculated by the moving average calculating section 104.

In a case where 80 steps/min, one step is equivalent to 750ms. Note here that, in a case where the sampling cycle is 20ms, 750/20 = 37.5. This shows that at least 37.5 samples per step are necessary.

The following table shows a relationship between the sampling cycle and the minimum required number of samples.

[Table 1]

| Sampling cycle (ms) | 20 | 30 | 40 | 50 | 60 | 70 | 80 |
|---|---|---|---|---|---|---|---|
| Minimum sample number | 37.5 | 25.0 | 18.8 | 15.0 | 12.5 | 10.7 | 9.4 |

The step counting section 116 determines, by use of the standard deviation σ obtained from the standard deviation calculating section 108, whether or not to carry out step counting.

In more detail, the step counting section 116 defines, by use of the standard deviation σ received from the standard deviation calculating section 108, a range of exclusion in which range there exist no upper peak value and no lower peak value for determination of whether or not to carry out step counting. Specifically, a range which is covered by the standard deviation σ from the moving average MA in an amplitude direction of the acceleration A (MA $\pm$ σ) is defined as the range of exclusion. An upper peak value UP and a lower peak value LP which are included in the range of exclusion serve as no upper peak value UP and no lower peak value LP for determination of whether or not to carry out step counting.

This point is described with reference to Fig. 12. Fig. 12 is a graph illustrating a case in which the step counting section 116 determines, by use of a standard deviation, whether or not to carry out step counting. Fig. 12 illustrates the waveform of the acceleration A, on which waveform a black dot denotes an upper peak value UP, a square outline with a blank inside denotes a lower peak value LP, and a cross (×) denotes a count determination point P. Further, in Fig. 12, a part surrounded by an ellipse is a surging part, and a part surrounded by a quadrangle is the range of exclusion.

The range of exclusion is the range of the standard deviation σ from the moving average MA at the count determination point P in the amplitude direction of the acceleration A (see Fig. 12). In a case where an upper peak value UP and a lower peak value LP of the surging part H10 are included in a range of exclusion J10, the step counting section 116 does not cause the upper peak value UP and the lower peak value LP of the surging part H10 to serve as the upper peak value UP and the lower peak value LP which are necessary for determination of whether or not to carry out step counting. Accordingly, the surging part H10 is not counted as a step, provided that the upper peak value UP and the lower peak value LP of the surging part H10 are included in the range of exclusion J10.

Next, the following description discusses, with reference to Fig. 13, a flow of a process in which the step counting section 116 determines whether or not to carry out step counting. Fig. 13 is a flowchart illustrating the flow of the process in which the step counting section 116 determines whether or not to carry out step counting. Note that the process which is described here and carried out by the step counting section 106 corresponds to the step detection process at S311 of Fig. 3 or Fig. 7.

First, the step counting section 116 determines whether or not a period between time $T_{UP}$ of an upper peak value UP received and time $T_{LP}$ of a lower peak value LP received ($T_{LP} - T_{UP}$) falls within a given range (y < $T_{LP}$ - $T_{UP}$ < δ) (S1301). In a case where γ < $T_{LP}$ - $T_{UP}$ < δ (YES at S1301), the step counting section 116 determines whether or not a difference between the upper peak value UP and the lower peak value LP is larger than a threshold ζ (UP - LP > ζ) (S1302). In a case where UP - LP > ζ (YES at S1302), the step counting section 116 instructs the standard deviation calculating section 108 to calculate a standard deviation σ (S1303), thereby obtaining the standard deviation σ calculated by the standard deviation calculating section 108 (S1304).

Then, the step counting section 116 determines whether or not the lower peak value LP is smaller than (MA - σ) and the upper peak value UP is larger than (MA + σ) (LP < (MA - σ) and UP > (MA + σ)) (S1305). In a case where LP < (MA - σ) and UP > (MA + σ) (YES at S1305), the step counting section 116 determines that walking is done as much as one step, thereby counting another step (S1306). Thereafter, the step detection process is ended, and the process proceeds to the step S312 or the step S702.

In contrast, in cases where (i) the period between the time $T_{UP}$ of the upper peak value UP received and the time $T_{LP}$ of the lower peak value LP received ($T_{LP} - T_{UP}$) does not fall within the given range (NO at S1301), (ii) the difference between the upper peak value UP and the lower peak value LP is not more than the threshold $\zeta$ (NO at S1302), and (iii) the requirement of LP < (MA - $\sigma$) and UP > (MA + $\sigma$) is not met (NO at S1305), the step counting process is then ended.

Note that the threshold $\zeta$ is a value such that, when a difference between an upper peak value and a lower peak value is smaller than $\zeta$, the upper peak value and the lower peak value are regarded as noises. The threshold $\zeta$, which is equivalent to or not less than 0.1G, is preferably set to a value which is suitable to be regarded as a noise by a sensor characteristic. For example, it is possible to set the threshold $\zeta$ so that $4096 \times (1/10) \times 0.1 \approx 41$.

Note that, since it is necessary to carry out a certain level of process with respect to calculation of a standard deviation, it is effective to decide a flow of a process so that whether or not a requirement employing a standard deviation is met is determined in a case where all the other requirements for step counting are met, as in the aforementioned flow of the process.

According to the arrangement, even if the ratio of the difference between the upper peak value UP and the moving average MA of the surging part to the difference between the lower peak value LP and the moving average MA of the surging part falls within the given range which serves as the requirement for counting of another step, it is possible to prevent counting of the surging part as a step.

Note that, since an amplitude of a surging part fluctuates in tandem with an amplitude of the whole waveform, it is preferable to cause a range of exclusion to fluctuate also in tandem with the whole waveform.

According to the arrangement, since a standard deviation $\sigma$ of the latest values of the acceleration A is used, it is possible to define an appropriate range of exclusion which range fluctuates in tandem with the latest waveform. Since the appropriate range of exclusion can be defined, it is possible to more accurately cause an upper peak value UP and a lower peak value LP of a surging part not to serve as the upper peak value UP and the lower peak value LP for determination of whether or not to carry out step counting. This makes it possible to more accurately determine whether or not to carry out step counting.

As described earlier, since a range of exclusion is defined not by use of an absolute value but by use of a standard deviation of the latest values of the acceleration A, it is unnecessary to change a program for defining the range of exclusion even if there occurs a change in input value per 1G due to, for example, a change of a sensor.

The present embodiment discusses a case in which both an upper peak value and a lower peak value of a surging part are included in a range of exclusion. In a case where only one of the upper peak value and the lower peak value is included in the range of exclusion, the other that is not included in the range of exclusion can serve as the upper peak value or the lower peak value for determination of whether or not to carry out step counting.

Further, in the case where only one of the upper peak value and the lower peak value is included in the range of exclusion, both the upper peak value and the lower peak value or none of the upper peak value and the lower peak value can serve as the upper peak value and the lower peak value for determination of whether or not to carry out step counting.

[Fourth Embodiment]

A further embodiment of the present invention is described below with reference to Figs. 14 through 18. Note that, for convenience, members having functions identical to those of the respective members illustrated in the respective First through Third Embodiments are given respective identical reference numerals, and a description of those members is omitted here.

The present embodiment is also directed to accurately carry out step counting in view of a surge in association with but not caused by walking.

In the First through Third Embodiments, when a surge occurs due to walking in which the acceleration A has a high amplitude, a surging part may be counted as a step.

This point is specifically described with reference to Fig. 14. Fig. 14 is a graph illustrating a case in which a surging part may be counted as a step.

Fig. 14 illustrates a waveform of an acceleration A, on which waveform a black dot denotes an upper peak value UP, a square outline with a blank inside denotes a lower peak value LP, and a cross ($\times$) denotes a count determination point P. Further, in Fig. 14, a part surrounded by an ellipse is a surging part. T401 shows a period between detection time of an upper peak value UP410 and detection time of a lower peak value LP411 at a count determination point P41. T402 shows a period between detection time of an upper peak value UP412 and detection time of a lower peak value LP413 at a count determination point P42.

In the First through Third Embodiments, whether or not to carry out step counting is determined depending on whether or not a period between an upper peak value UP and a lower peak value LP ($T_{LP} - T_{UP}$) falls within a given range. For this reason, in a case where the period T402 between the detection time of the upper peak value UP412 and the detection time of the lower peak value LP413 falls within the given range, a surging part may be counted as a step at the count determination point P42.

In the present embodiment, as a requirement for a period for use in step counting, it is determined not only whether or not the period between the upper peak value UP and the lower peak value LP ($T_{LP}$ - $T_{UP}$) falls within the given range but also whether or not a period between detection time of a lower peak value detected last time when another step was counted and detection time of an upper peak value detected this time is larger than a threshold.

Specifically, the present embodiment is different from the First Embodiment in that the step counting section 106 of the portable terminal 1 is replaced with a step counting section 117 (not illustrated). The step counting section 117 also determines, as a requirement for step counting, whether or not a period between detection time of a lower peak value detected last time when another step was counted and detection time of an upper peak value detected this time is larger than a threshold.

This point is described in more detail with reference to Fig. 15. Fig. 15 is a graph illustrating a case in which whether or not to carry out step counting is determined by use of a requirement under which a period between detection time of a lower peak value used for previous determination of step counting and detection time of an upper peak value detected this time is not less than a threshold.

Similarly to Fig. 14, Fig. 15 illustrates a waveform of an acceleration A, on which waveform a black dot denotes an upper peak value UP, a square outline with a blank inside denotes a lower peak value LP, and a cross ($\times$) denotes a count determination point P. Further, in Fig. 15, a part surrounded by an ellipse is a surging part. T401 shows a period between detection time of an upper peak value UP410 and detection time of a lower peak value LP411 at a count determination point P41. T402 shows a period between detection time of an upper peak value UP412 and detection time of a lower peak value LP413 at a count determination point P42. T403 shows a period between detection time of an upper peak value UP412 at the count determination point P42 and the detection time of the lower peak value LP411 at the count determination point P41 which is a previous point at which another step was counted. T404 shows a period between detection time of an upper peak value UP414 at a count determination point P43 and the detection time of the lower peak value LP411 at the count determination point P41 which is a previous point at which another step was counted.

In the present embodiment, in the determination of whether or not to carry out step counting, the step counting section 117 further determines whether or not a period between time $T_{UP}$ of an upper peak value UP detected this time and time $T_{WLP}$ of a lower peak value LP detected last time another step was counted is larger than a threshold $\eta$ ($T_{WLP}$ - $T_{UP}$ > $\eta$).

Note that the threshold $\eta$ is set to a minimum time interval which is considered to be appropriate, in human walking, for a time interval between a previous step and a subsequent step. In the present embodiment, the threshold $\eta$ is set to 100ms.

This can prevent counting of a subsequent step as a step in a case where a time interval between a previous step and the subsequent step is too short to be regarded as human walking.

In the case of Fig. 15, in a case where T403 is not more than the threshold $\eta$ at the count determination point P42, no step counting is carried out even if the other requirements for counting of another step are met. In a case where T404 is larger than the threshold $\eta$ at the count determination point P43 and the other requirements for counting of another step are also met, step counting is carried out.

Next, the following description discusses, with reference to Fig. 16, a flow of a process in which the step counting section 117 determines whether or not to carry out step counting. Fig. 16 is a flowchart illustrating the flow of the process in which the step counting section 117 determines whether or not to carry out step counting. Note that the process which is described here and carried out by the step counting section 117 corresponds to the step detection process at S311 of Fig. 3 or Fig. 7.

First, the step counting section 117 determines whether or not a difference between an upper peak value UP and a lower peak value LP which have been received is larger than a threshold $\zeta$ (UP - LP > $\zeta$) (S1601). In a case where UP - LP > $\zeta$ (YES at S1601), the step counting section 117 subsequently determines whether or not a ratio of a difference between the upper peak value UP and a corresponding moving average $MA_{UP}$ to a difference between the lower peak value LP and a corresponding moving average $MA_{UP}$ falls within a threshold range of $1/\beta$ < {(UP - $MA_{UP}$)/($MA_{LP}$ - LP)} < $\beta$ (S1602). In a case where $1/\beta$ < {(UP - $MA_{UP}$)/($MA_{LP}$ - LP)} < $\beta$ (YES at S 1602), the step counting section 117 determines whether or not a period between the upper peak value UP and the lower peak value LP ($T_{LP}$ - $T_{UP}$) falls within a given range of $\gamma$ < $T_{LP}$ - $T_{UP}$ < $\delta$ (S1603).

In a case where $\gamma$ < $T_{LP}$ - $T_{UP}$ < $\delta$ (YES at S 1603), the step counting section 117 determines whether or not the period between the time $T_{WLP}$ of the lower peak value detected last time another step was counted and the time $T_{UP}$ of the upper peak value UP detected this time is larger than the threshold $\eta$ ($T_{UP}$ - $T_{WLP}$ > $\eta$) (S 1604). In a case where $T_{UP}$ - $T_{WLP}$ > $\eta$ (YES at 1604), the step counting section 117 determines that walking is done as much as one step, thereby counting another step (S1605). Thereafter, the step detection process is ended, and the process proceeds to the step S3 12 or the step S702.

In contrast, in cases where (i) the difference between the upper peak value UP and the lower peak value LP which have been received is not more than the threshold $\zeta$ (NO at S1302), (ii) the ratio of the difference between the upper peak value UP and the corresponding moving average $MA_{UP}$ to the difference between the lower peak value LP and the corresponding moving average $MA_{UP}$ does not fall within the threshold range (NO at S1602), and (iii) a period between the time $T_{UP}$ of the upper peak value UP and time $T_{LP}$ of the lower peak value LP ($T_{LP}$ - $T_{UP}$) does not fall within the given range (NO at S 1603), and (iv) the period between the time $T_{WLP}$ of the lower peak value detected last time another step was counted and the time $T_{UP}$ of the upper peak value UP detected this time is not more than the threshold η (NO at S1604), the step counting process is then ended.

In the present embodiment, the requirement of $T_{UP}$ - $T_{WLP}$ > η serves as a requirement for counting of another step. A requirement of $T_{UP}$ - $T_{WLP}$ ≥ η can also serve as a requirement for counting of another step.

According to the arrangement, it is possible to prevent counting of a subsequent step in a time interval from previous counting of another step which time interval is too long or too short to be regarded as human walking. This can prevent a surge, which occurs in a short period after the previous counting of another step, from being counted as a step.

Further, it is also possible to combine the step detection process of the present embodiment carried out by the step counting section 117 with the step detection process of the Third Embodiment carried out by the step counting section 116. This point is described below with reference to Fig. 17. Fig. 17 is a flowchart illustrating a flow of a process in which a step counting section 118 (not illustrated) determines whether or not to carry out step counting. Note that the process which is described here and carried out by the step counting section 118 corresponds to the step detection process at S311 of Fig. 3 or Fig. 7.

First, the step counting section 118 determines whether or not a period between time $T_{UP}$ of an upper peak value UP received and time $T_{LP}$ of a lower peak value LP received ($T_{LP}$ - $T_{UP}$) falls within the given range ($\gamma < T_{LP}$ - $T_{UP}$ < δ) (S1701) (see Fig. 17). In a case where $\gamma < T_{LP}$ - $T_{UP}$ < δ (YES at S1701), the step counting section 118 determines whether or not a period between time $T_{WLP}$ of a lower peak value detected last time another step was counted and the time $T_{UP}$ of the upper peak value UP detected this time is larger than a threshold η ($T_{UP}$ - $T_{WLP}$ > η) (S1702). In a case where $T_{UP}$ - $T_{WLP}$ > η (YES at S1702), the step counting section 118 determines whether or not a difference between the upper peak value UP and the lower peak value LP is larger than a threshold ζ (UP - LP > ζ) (S1703). In a case where UP - LP > ζ (YES at S1703), the step counting section 118 instructs a standard deviation calculating section 108 to calculate a standard deviation σ (S1704), thereby obtaining the standard deviation σ calculated by the standard deviation calculating section 118 (S1705).

Then, the step counting section 118 determines whether or not the lower peak value LP is smaller than (MA - σ) and the upper peak value UP is larger than (MA + σ) (LP < (MA - σ) and UP > (MA + σ)) (S1706). In a case where LP < (MA - σ) and UP > (MA + σ) (YES at S1706), the step counting section 118 determines that walking is done as much as one step, thereby counting another step (S1707). Thereafter, the step detection process is ended, and the process proceeds to the step S312 or the step S702.

In contrast, in cases where (i) the period between the time $T_{UP}$ of the upper peak value UP received and the time $T_{LP}$ of the lower peak value LP received ($T_{LP}$ - $T_{UP}$) does not fall within the given range (NO at S1701), (ii) the period between the time $T_{WLP}$ of the lower peak value detected last time another step was counted and the time $T_{UP}$ of the upper peak value UP detected this time is not more than the threshold η (NO at S1702), (iii) the difference between the upper peak value UP and the lower peak value LP is not more than the threshold ζ (NO at S1703), and (iv) the requirement of LP < (MA - σ) and UP > (MA + σ) is not met (NO at S1706), the step counting process is then ended.

[Fifth Embodiment]

A still further embodiment of the present invention is described below with reference to Figs. 18 through 22. Note that, for convenience, members having functions identical to those of the respective members illustrated in the respective First through Fourth Embodiments are given respective identical reference numerals, and a description of those members is omitted here.

According to the arrangement described in the Third Embodiment, another step that should be counted may not be counted. This point is described below with reference to Fig. 18. Fig. 18 is a graph illustrating a case in which another step that should be counted may not be counted when a range of exclusion is defined by use of a standard deviation σ of an acceleration A.

Fig. 18 illustrates a waveform of an acceleration A, on which waveform a black dot denotes an upper peak value UP, a square outline with a blank inside denotes a lower peak value LP, and a cross (×) denotes a count determination point P. Further, in Fig. 18, a part surrounded by a quadrangle is the range of exclusion, and a part surrounded by an ellipse is a part in which step counting that should be carried out is not carried out.

According to the arrangement of the Third Embodiment, another step that should be counted may not be counted in a case where the waveform of the acceleration A has initial high amplitudes followed by low amplitudes (see Fig. 18).

This is because of the following reason. In a part of the waveform which part has a high amplitude has a large standard deviation σ. Accordingly, the range of exclusion also becomes larger. Then, a count determination point P of the waveform whose amplitude is low just after its high amplitude also has a large standard deviation σ since the count determination point P includes a value of the acceleration A of the part which has a high amplitude. Accordingly, the range of exclusion also becomes larger, and an upper peak value UP and a lower peak value LP which are obtained at the count determination point P of the waveform whose amplitude is low may be included in the range of exclusion.

In a case where an upper peak value UP 801 and a lower peak value LP 802 at a count determination point P811 are included in a range of exclusion H81 (see Fig. 18), no step counting is carried out in the arrangement of the Third Embodiment.

In view of the circumstances, in the present embodiment, step counting is carried out not only in a case where a difference between an upper peak value and a lower peak value exceeds a threshold but also in a case where the difference does not exceed the threshold but the other requirements under which step counting should be carried out are met.

Specifically, the present embodiment is different from the Third Embodiment in that the step counting section 116 of the Third Embodiment is replaced with a step counting section 119 (not illustrated). The following description discusses a method in which the step counting section 119 determines whether or not to carry out step counting.

A case in which the step counting section 119 carries out step counting is described below with reference to Fig. 19. Fig. 19 is a graph illustrating a case in which the step counting section 119 carries out step counting. Fig. 19 illustrates a waveform of an acceleration A, on which waveform a black dot denotes an upper peak value UP, a square outline with a blank inside denotes a lower peak value LP, and a cross ($\times$) denotes a count determination point P. Further, in Fig. 18, a part surrounded by a quadrangle is the range of exclusion, and a range shown in a straight line defined by triangles is a threshold $\alpha$. Note that, in a case where a difference between a lower peak value and an upper peak value exceeds the threshold $\alpha$, the difference is regarded as human walking irrespective of the other requirements. In the present embodiment, the threshold $\alpha$ is equivalent to 0.35G, as described earlier.

The step counting section 119 carries out step counting in a case where, at the count determination point P, a period between time $T_{UP}$ of the upper peak value UP and time $T_{LP}$ of the lower peak value LP ($T_{LP} - T_{UP}$) falls within a given range and a difference between the upper peak value UP and the lower peak value LP is larger than the threshold $\alpha$. In Fig. 19, in a case where the period between the time $T_{UP}$ of the upper peak value UP and the time $T_{LP}$ of the lower peak value LP ($T_{LP} - T_{UP}$) falls within the given range, the difference between the upper peak value UP and the lower peak value LP, which difference corresponds to a step count of 4 exceeds the threshold $\alpha$, is counted as a step.

In contrast, in a case where, at the count determination point P, the period between the time $T_{UP}$ of the upper peak value UP and the time $T_{LP}$ of the lower peak value LP ($T_{LP} - T_{UP}$) falls within the given range but the difference between the upper peak value UP and the lower peak value LP does not exceed the threshold $\alpha$, the step counting section 119 carries out determination of the following two requirements, thereby determining whether or not to carry out step counting. The first requirement is whether or not the difference between the upper peak value UP and the lower peak value LP is larger than a threshold $\zeta$. The second requirement is whether or not the lower peak value LP is smaller than (MA - $\sigma$) and the upper peak value UP is larger than (MA + $\sigma$).

The following description discusses, with reference to Fig. 20, a flow of a process in which the step counting section 119 determines whether or not to carry out step counting. Fig. 20 is a flowchart illustrating the flow of the process in which the step counting section 119 determines whether or not to carry out step counting. Note that the process which is described here and carried out by the step counting section 119 corresponds to the step detection process at S311 of Fig. 3 or Fig. 7.

The step counting section 119 determines whether or not the period between the time $T_{UP}$ of the upper peak value UP received and the time $T_{LP}$ of the lower peak value LP received ($T_{LP} - T_{UP}$) falls within the given range ($\gamma < T_{LP} - T_{UP} < \delta$) (S2001) (see Fig. 20). In a case where $\gamma < T_{LP} - T_{UP} < \delta$ (YES at S2001), the step counting section 119 determines whether or not the difference between the upper peak value UP and the lower peak value LP is larger than the threshold $\alpha$ (UP - LP > $\alpha$) (S2002). In a case where UP - LP > $\alpha$ (YES at S2002), the step counting section 119 determines that walking is done as much as one step, thereby counting another step (S2007).

In contrast, in a case where UP - LP $\leq \alpha$ (NO at S2002), the step counting section 119 determines whether or not the difference between the upper peak value UP and the lower peak value LP is larger than the threshold $\zeta$ (UP - LP > $\zeta$) (S2003). In a case where UP - LP > $\zeta$ (YES at S2003), the step counting section 119 instructs a standard deviation calculating section 108 to calculate a standard deviation $\sigma$ (S2004), thereby obtaining the standard deviation $\sigma$ calculated by the standard deviation calculating section 108 (S2005).

Then, the step counting section 119 determines whether or not the lower peak value LP is smaller than (MA - $\sigma$) and the upper peak value UP is larger than (MA + $\sigma$) (LP < (MA - $\sigma$) and UP > (MA + $\sigma$)) (S2006). In a case where LP < (MA - $\sigma$) and UP > (MA + $\sigma$) (YES at S2006), the step counting section 119 determines that walking is done as much as one step, thereby counting another step (S2007). Thereafter, the process proceeds to the step S312 or the step S702.

In contrast, in cases where (i) the period between the time $T_{UP}$ of the upper peak value UP received and the time $T_{LP}$ of the lower peak value LP received ($T_{LP} - T_{UP}$) does not fall within the given range (NO at S2001), (ii) the difference between the upper peak value UP and the lower peak value LP is not more than the threshold $\zeta$ (NO at S2003),

and (iii) the requirement of LP < (MA - σ) and UP > (MA + σ) is not met (NO at S2006), the step counting process is then ended.

According to the arrangement, it is possible to accurately carry out step counting even in a case where the waveform of the acceleration A has an amplitude which follows a high amplitude and is so high that the waveform is counted as a step. Further, it is possible to prevent a surge which should not be counted as a step from being counted as a step.

It is also possible to combine, with the present embodiment, the arrangement described in the Fourth Embodiment. In this case, the following description discusses, with reference to Fig. 21, a flow of a process in which the step counting section 119 determines whether or not to carry out step counting. Fig. 21 is a flowchart illustrating the flow of the process in which the step counting section 119 determines whether or not to carry out step counting.

The step counting section 119 determines whether or not the period between the time $T_{UP}$ of the upper peak value UP received and the time $T_{LP}$ of the lower peak value LP received ($T_{LP} - T_{UP}$) falls within the given range ($\gamma < T_{LP} - T_{UP} < \delta$) (S2101) (see Fig. 21). In a case where $\gamma < T_{LP} - T_{UP} < \delta$ (YES at S2101), the step counting section 119 determines whether or not a period between time $T_{WLP}$ of a lower peak value detected last time another step was counted and the time $T_{UP}$ of the upper peak value UP detected this time is larger than the threshold η ($T_{UP} - T_{WLP} > \eta$) (S2102).

In a case where $T_{UP} - T_{WLP} > \eta$ (YES at S2102), the step counting section 119 determines whether or not the difference between the upper peak value UP and the lower peak value LP is larger than the threshold α (UP - LP > α) (S2103). In a case where UP - LP > α (YES at S2103), the step counting section 119 determines that walking is done as much as one step, thereby counting another step (S2108).

In contrast, in a case where UP - LP ≤ α (NO at S2103), the step counting section 119 determines whether or not the difference between the upper peak value UP and the lower peak value LP is larger than the threshold ζ (UP - LP > ζ) (S2104). In a case where UP - LP > ζ (YES at S2104), the step counting section 119 instructs the standard deviation calculating section 108 to calculate the standard deviation σ (S2105), thereby obtaining the standard deviation σ calculated by the standard deviation calculating section 108 (S2106).

Then, the step counting section 119 determines whether or not the lower peak value LP is smaller than (MA - σ) and the upper peak value UP is larger than (MA + σ) (LP < (MA - σ) and UP > (MA + σ)) (S2107). In a case where LP < (MA - σ) and UP > (MA + σ) (YES at S2107), the step counting section 119 determines that walking is done as much as one step, thereby counting another step (S2108). Thereafter, the process proceeds to the step S312 or the step S702.

In contrast, in cases where (i) the period between the time $T_{UP}$ of the upper peak value UP received and the time $T_{LP}$ of the lower peak value LP received ($T_{LP} - T_{UP}$) does not fall within the given range (NO at S2101), (ii) the period between the time $T_{WLP}$ of the lower peak value detected last time another step was counted and the time $T_{UP}$ of the upper peak value UP detected this time is not more than the threshold η (NO at S2103), (iii) the difference between the upper peak value UP and the lower peak value LP is not more than the threshold ζ (NO at S2104), and (iv) the requirement of LP < (MA - σ) and UP > (MA + σ) is not met (NO at S2107), the step counting process is then ended.

According to the arrangement, it is possible to further prevent counting of a subsequent step in a time interval from previous counting of another step which time interval is too long or too short to be regarded as human walking. This can prevent a surge, which occurs in a short period after the previous counting of another step, from being counted as a step.

The present invention is not limited to the description of the embodiments above, but may be altered by a skilled person within the scope of the claims. An embodiment based on a proper combination of technical means disclosed in different embodiments is encompassed in the technical scope of the present invention.

Finally, each of the blocks of the portable terminals 1, 2, and 3, especially the quantizing section 101, the offset correction section 102, and the scalarizing section 103, the moving average calculating section 104, the peak detecting section 105, and the step counting section 106 (107, 116, 117, 118, or 119) which are included in the control section 10 can be configured by hardware logic or realized by a software by use of a CPU as described below.

Namely, each of the portable terminals 1, 2, and 3 includes a CPU which carries out an instruction from a control program for realizing each of the functions, a ROM (read only memory) in which the program is stored, a RAM

(random access memory) which decompresses the program, a storage (recording medium) (e.g., a memory in which the program and various data are stored). It is also possible to attain the object of the present invention by supplying each of the portable terminals 1, 2, and 3 with a recording medium in which program codes (an executable program, an intermediate code program, and a source program) of the control program of each of the portable terminals 1, 2, and 3 for realizing each of the functions are computer-readably recorded and then causing a computer (or a CPU or an MPU (microprocessor unit)) of each of the portable terminals 1, 2, and 3 to read out and carry out the program codes recorded in the recording medium.

Examples of the recording medium include: (i) tapes such as a magnetic tape and a cassette tape, (ii) disks including magnetic disks such as a floppy (registered trademark) disk and a hard disk and optical disks such as a CD-ROM (compact disc read-only memory), an MO (magnetooptical), an MD (Mini Disc), a DVD (digital video disk), and a CD-R (CD Recordable), (iii) cards such as an IC card (including a memory card) and an optical card, and (iv) semiconductor memories such as a mask ROM, an EPROM (erasable programmable read-only memory), an EEPROM (electrically erasable and programmable read-only memory), and a flash ROM.

Further, it is possible to arrange each of the portable terminals 1, 2, and 3 to be connectable to a communication network, via which the program codes can be supplied. The communication network is not particularly limited. Examples of the communication network include: the Internet, the Intranet, the Extranet, LAN (local area network), ISDN (integrated services digital network), VAN (value-added network), a CATV (community antenna television) communication network, a virtual private network, a telephone circuit network, a mobile telecommunications network, and a satellite communications network. A transmission medium constituting the communication network is not particularly limited. Examples of the transmission medium include: wired transmission mediums such as IEEE (institute of electrical and electronic engineers) 1394, USB, a power-line carrier, a cable TV circuit, a telephone line, and ADSL (asynchronous digital subscriber loop); and wireless transmission mediums such as infrared communication systems such as IrDA (infrared data association) and a remote control, Bluetooth (registered trademark), 802.11 wireless communication system, HDR (high data rate), a mobile phone network, a satellite circuit, and a digital terrestrial network. Note that the present invention can also be realized in a form of a computer data signal in which the program codes are embodied by an electronic transmission and which is embedded in carrier waves.

As described earlier, the body movement measuring device in accordance with the present invention is preferably arranged to further include: standard deviation calculating means for obtaining, at every given time, the combined value obtained by the scalarizing means, and calculating a standard deviation of the combined value obtained from a given time point up to present time; and average calculating means for obtaining, at every given time, the combined value obtained by the scalarizing means, and calculating an average of the combined value obtained from a given time point up to present time, the step counting means counting another step when the upper peak value exceeds a sum of the average and the standard deviation and the lower peak value falls below a difference between the average and the standard deviation.

Note here that a given time point refers to a time point at which a period between the given time point and present time is as long as possible so that a vibration generated during walking due to a reason different from walking is less influential to an acceleration, but as short as possible so that a change in direction of an acceleration due to, for example, a change in direction of the device can be followed.

According to the arrangement, standard deviation calculating means obtains, at every given time, the combined value obtained by the scalarizing means, and calculates a standard deviation of the combined value obtained from a given time point up to present time. Furthermore, average calculating means obtains, at every given time, the combined value obtained by the scalarizing means, and calculates an average of the combined value obtained from a given time point up to present time. Moreover, the step counting means counts another step when the upper peak value exceeds a sum of the average and the standard deviation and the lower peak value falls below a difference between the average and the standard deviation.

According to this, another step is counted in a case where a difference between an upper peak value and a lower peak value does not exceed the given value but the upper peak value exceeds a sum of the average and the standard deviation and the lower peak value falls below a difference between the average and the standard deviation. Therefore, it is possible to prevent a case in which another step that should be counted is not counted since a difference between an upper peak value and a lower peak value does not exceed the given value.

For example, in a case where a difference between an upper peak value and a lower peak value does not exceed the given value though walking is done, another step is counted as long as the upper peak value and the lower peak value meet the aforementioned requirement. Accordingly, it is possible to prevent a case in which no step counting is carried out in all cases where a difference between an upper peak value and a lower peak value does not exceed the given value.

When an upper peak value falls below a sum of the average and the standard deviation or a lower peak value exceeds a difference between the average and the standard deviation, counting of another step is not carried out. Therefore, it is possible to prevent counting of another step in a case where there exist an upper peak value and a lower peak value though no walking is done.

No step counting is carried out, for example, in a case where a surge or the like produces an upper peak value and a lower peak value but the upper peak value falls below a sum of the average and the standard deviation or the lower peak value exceeds a difference between the average and the standard deviation.

Accordingly, it is possible to prevent counting of another step in the case of a surge or the like which should not be counted as a step. Note here that a surge refers to a case in which a relationship between an upper peak value and a lower peak value which relationship should not be counted as a step is similar to a relationship between an upper peak value and lower peak value which relationship should be counted as a step.

The body movement measuring device in accordance with the present invention is preferably arranged such that the step counting means counts another step when the following conditions are all met: (i) a period between (a) detection time of a lower peak value detected last time when another step was counted and (b) detection time of an upper peak value detected this time does not fall within a given range; and (ii) the upper peak value detected this time exceeds the sum of the average and the standard deviation and a lower peak value detected this time falls below the difference between the average and the standard deviation.

Note here that, the given range is such a range that, in a case where a period between (a) detection time of a lower peak value detected last time when another step was counted and (b) detection time of an upper peak value detected this time falls within the given range, what is detected is difficult to be regarded as human walking.

According to the arrangement, the step counting means counts another step when the following conditions are all met: (i) a period between (a) detection time of a lower peak value detected last time when another step was counted and (b) detection time of an upper peak value detected this time does not fall within a given range; and (ii) the upper peak value detected this time exceeds the sum of the average and the standard deviation and a lower peak value detected this time falls below the difference between the average and the standard deviation.

According to this, another step is not counted in a case where a period between (a) detection time of a lower peak value detected last time when another step was counted and (b) detection time of an upper peak value detected this time falls within the given range. This can prevent counting of a subsequent step in an interval between a previous step and a subsequent step which interval is too long or too short to be regarded as human walking.

Further, another step is not counted in a case where a difference between detection time of an upper peak value of a surging part and detection time of a lower peak value detected last time when another step was counted does not fall within the given range. This can prevent counting of another step from being carried out with respect to a surging part in which an upper peak value exists in the given range from detection time of a lower peak value detected last time when another step was counted.

The body movement measuring device in accordance with the present invention is preferably arranged to further include: average calculating means for obtaining, at every given time, the combined value obtained by the scalarizing means, and calculating an average of the combined value obtained from a given time point up to present time, the step counting means finding (i) a difference between the upper peak value and an average of the combined value obtained from the given time point to when the combined value reaches the upper peak value and (ii) a difference between the lower peak value and an average of the combined value obtained from the given time point to when the combined value reaches the lower peak value, wherein the step counting means counts another step when a ratio between the differences (i) and (ii) falls within a given range and a difference between the upper peak value and the lower peak value exceeds a given value.

Note here that a given time point refers to a time point at which a period between the given time point and present time is as long as possible so that a vibration generated during walking due to a reason different from walking is less influential to an acceleration, but as short as possible so that a change in direction of an acceleration due to, for example, a change in direction of the device can be followed.

Note also that a given range is such a range that when the ratio between the differences falls within the given range, the ratio is not considered to be unreasonable in consideration of the reality of human walking.

According to the arrangement, the step counting means counts another step when a ratio between (i) a difference between the upper peak value and an average of the combined value obtained from the given time point to when the combined value reaches the upper peak value and (ii) a difference between the lower peak value and an average of the combined value obtained from the given time point to when the combined value reaches the lower peak value falls within the given range and a difference between the upper peak value and the lower peak value exceeds the given value.

This can prevent counting of another step merely by the fact that a change in acceleration due to some external factor causes a difference between an upper peak value and a lower peak value to exceed the given value though no walking is done.

The body movement measuring device in accordance with the present invention can be arranged to further include: a median calculating means for calculating a median of the upper peak value and the lower peak value which have been detected by the peak value detecting means, the step counting means finding (i) a difference between the upper peak value and the median and (ii) a difference between the lower peak value and the median, wherein the step counting means counts another step when a ratio between the differences (i) and (ii) falls within a given range and the difference between the upper peak value and the lower peak value exceeds the given value.

Note here that a given range is such a range that when the ratio between the differences falls within the given range, the ratio is not considered to be unreasonable in consideration of the reality of human walking.

According to the arrangement, the step counting means counts another step when a ratio between (i) a difference between the upper peak value and the median and (ii) a difference between the lower peak value and the median falls within a given range and the difference between the upper peak value and the lower peak value exceeds the given value.

This can prevent counting of another step merely by the fact that a change in acceleration due to some external factor causes a difference between an upper peak value and a lower peak value to exceed the given value though no walking is done. Accordingly, it is possible to accurately measure body movements.

The body movement measuring device in accordance with the present invention is preferably arranged such that the step counting means does not count another step when a period between detection time of the upper peak value and detection time of the lower peak value does not fall within a given range.

Note here that a given range is such a range that what is detected can be regarded as human walking in a case where there exists a period in the given range.

According to the arrangement, another step is not counted when a period between detection time of the upper peak value and detection time of the lower peak value does not fall within a given range.

According to this, in a case where the period is too long or too short to be regarded as human walking, it is possible to prevent counting of another step.

The body movement measuring device in accordance with the present invention can be arranged such that: the step counting means carries out determination of whether or not to count another step, for every one cycle of a waveform formed by the combined value; and in a case where the step counting means does not count another step as a result of the determination, the step counting means carries out the determination by use of one of upper peak values and one of lower peak values in a period of a given number of cycles to a current cycle.

Note here that the given number of cycles refers to any range of cycles between last time when another step was counted and this time when whether or not to count another step is determined.

According to the arrangement, in a case where the step counting means does not count another step as a result of the determination, the step counting means carries out the determination by use of one of upper peak values and one of lower peak values in a period of a given number of cycles to a current cycle.

This can prevent a case in which another step that should be counted is not counted since a change in acceleration during walking due to some external factor produces a plurality of upper peak values and lower peak values but differences between the respective upper peak values and lower peak values do not exceed a given value. Accordingly, it is possible to accurately measure body movements.

It is possible to yield the aforementioned effect also in a case where the given number of cycles is one cycle before the current cycle.

Note that it is possible to cause a computer to realize a body movement measuring device mentioned above. In this case, (i) a body movement measuring device control program for causing the computer to realize the body movement measuring device by causing the computer to operate as each means mentioned above and (ii) a computer-readable recording medium in which the body movement measuring device control program is recorded are both encompassed in the scope of the present invention.

As described earlier, a body movement measuring device in accordance with the present invention includes: an acceleration detecting section for detecting accelerations in a plurality of directions; scalarizing means for combining the accelerations detected by the acceleration detecting section, so as to obtain a combined value, and then scalarizing the combined value; peak value detecting means for obtaining, at every given time, the combined value obtained by the scalarizing means, and detecting an upper peak value and a lower peak value of the combined value thus obtained; and step counting means for counting another step when a difference between the upper peak value and the lower peak value which have been detected by the peak value detecting means exceeds a given value.

A method in accordance with the present invention for controlling a body movement measuring device includes the steps of: (a) combining the accelerations detected by the acceleration detecting section, so as to obtain a combined value, and then scalarizing the combined value thus obtained; (b) sampling, at every given time, the combined value obtained in the step (a), and detecting an upper peak value and a lower peak value of the combined value thus sampled; and (c) counting another step when a difference between the upper peak value and the lower peak value which have been detected in the step (b) exceeds a given value.

According to this, it is possible to accurately carry out step counting even if (i) an error in a sensor for detecting an acceleration causes the sensor to output a larger or smaller value than a value which is supposed to be outputted and/or (ii) a change in offset voltage corresponding to each sensor causes a value corrected by the offset voltage to be larger or smaller than a value which is supposed to be set.

Industrial Applicability

The present invention is suitable for portable devices such as a pedometer and a portable terminal having a pedometer function since the present invention makes it possible to appropriately carry out step counting even if there occur(s) an error in a body movement sensor and/or an error due to a change in offset voltage.

**Claims**

**1.** A body movement measuring device comprising:

an acceleration detecting section for detecting accelerations in a plurality of directions;
scalarizing means for combining the accelerations detected by the acceleration detecting section, so as to obtain a combined value, and then scalarizing the combined value;
peak value detecting means for obtaining, at every given time, the combined value obtained by the scalarizing means, and detecting an upper peak value and a lower peak value of the combined value thus obtained; and
step counting means for counting another step when a difference between the upper peak value and the lower peak value which have been detected by the peak value detecting means exceeds a given value.

2. The body movement measuring device as set forth in claim 1, further comprising:

standard deviation calculating means for obtaining, at every given time, the combined value obtained by the scalarizing means, and calculating a standard deviation of the combined value obtained from a given time point up to present time; and
average calculating means for obtaining, at every given time, the combined value obtained by the scalarizing means, and calculating an average of the combined value obtained from a given time point up to present time, the step counting means counting another step when the upper peak value exceeds a sum of the average and the standard deviation and the lower peak value falls below a difference between the average and the standard deviation.

3. The body movement measuring device as set forth in claim 2, wherein the step counting means counts another step when the following conditions are all met: (i) a period between (a) detection time of a lower peak value detected last time when another step was counted and (b) detection time of an upper peak value detected this time does not fall within a given range; and (ii) the upper peak value detected this time exceeds the sum of the average and the standard deviation and a lower peak value detected this time falls below the difference between the average and the standard deviation.

4. The body movement measuring device as set forth in claim 1, further comprising:

average calculating means for obtaining, at every given time, the combined value obtained by the scalarizing means, and calculating an average of the combined value obtained from a given time point up to present time, the step counting means finding (i) a difference between the upper peak value and an average of the combined value obtained from the given time point to when the combined value reaches the upper peak value and (ii) a difference between the lower peak value and an average of the combined value obtained from the given time point to when the combined value reaches the lower peak value, wherein the step counting means counts another step when a ratio between the differences (i) and (ii) falls within a given range and a difference between the upper peak value and the lower peak value exceeds a given value.

5. The body movement measuring device as set forth in claim 1, further comprising:

a median calculating means for calculating a median of the upper peak value and the lower peak value which have been detected by the peak value detecting means,
the step counting means finding (i) a difference between the upper peak value and the median and (ii) a difference between the lower peak value and the median, wherein the step counting means counts another step when a ratio between the differences (i) and (ii) falls within a given range and the difference between the upper peak value and the lower peak value exceeds the given value.

6. The body movement measuring device as set forth in any one of claims 1 through 5, wherein the step counting means does not count another step when a period between detection time of the upper peak value and detection time of the lower peak value does not fall within a given range.

7. The body movement measuring device as set forth in any one of claims 1 through 6, wherein:

the step counting means carries out determination of whether or not to count another step, for every one cycle of a waveform formed by the combined value; and
in a case where the step counting means does not count another step as a result of the determination, the step counting means carries out the determination by use of one of upper peak values and one of lower peak values in a period of a given number of cycles to a current cycle.

8. The body movement measuring device as set forth in claim 7, wherein the given number of cycles is one cycle before the current cycle.

9. The body movement measuring device as set forth in any one of claims 1 through 8, wherein:

the acceleration detecting section is an acceleration sensor for detecting accelerations in directions of three axes which cross each other orthogonally;
said body movement measuring device further includes quantizing means for quantizing outputs from the respective three axes of the acceleration sensor; and
the scalarizing means performs the scalarization for the three outputs quantized by the quantizing means.

10. The body movement measuring device as set forth in claim 9, wherein:

the acceleration sensor has a sensitivity which is a tenth of a power supply voltage per 1G;
the quantizing means is an AD converter which has a quantization bit rate of 12; and
the given value used by the step counting means is 143.

11. The body movement measuring device as set forth in claim 10, wherein the given range recited in claim 4 or 5 ranges from one-fourth to four.

12. The body movement measuring device as set forth in any one of claims 1 through 11, wherein the given range recited in claim 3 is not more than 100ms.

13. The body movement measuring device as set forth in any one of claims 1 through 12, wherein the given range recited in claim 6 ranges from 100ms to 500ms.

14. The body movement measuring device as set forth in any one of claims 1 through 13, wherein the given time is 30ms.

15. A mobile phone comprising a body movement measuring device recited in any one of claims 1 through 14.

16. A body movement measuring device control program for causing a body movement measuring device recited in any one of claims 1 through 14 to operate, said body movement measuring device control program causing a computer to function as each means of the body movement measuring device.

17. A computer-readable recording medium in which the body movement measuring device control program recited in claim 16 is recorded.

18. A method for controlling a body movement measuring device including an acceleration detecting section for detecting accelerations in a plurality of directions,
said method comprising the steps of:

(a) combining the accelerations detected by the acceleration detecting section, so as to obtain a combined value, and then scalarizing the combined value thus obtained;
(b) sampling, at every given time, the combined value obtained in the step (a), and detecting an upper peak value and a lower peak value of the combined value thus sampled; and
(c) counting another step when a difference between the upper peak value and the lower peak value which have been detected in the step (b) exceeds a given value.

**Amended claims under Art. 19.1 PCT**

1. A body movement measuring device comprising:

an acceleration detecting section for detecting accelerations in a plurality of directions;
scalarizing means for combining the accelerations detected by the acceleration detecting section, so as to obtain a combined value, and then scalarizing the combined value;
peak value detecting means for obtaining, at every given time, the combined value obtained by the scalarizing means, and detecting an upper peak value and a lower peak value of the combined value thus obtained; and

step counting means for counting another step when a difference between the upper peak value and the lower peak value which have been detected by the peak value detecting means exceeds a given value.

**2.** Amended) The body movement measuring device as set forth in claim 1, wherein the upper peak value and the lower peak value are located adjacently on an time axis.

**3.** Amended) The body movement measuring device as set forth in claim 1, further comprising:

average calculating means for obtaining, at every given time, the combined value obtained by the scalarizing means, and calculating an average of the combined value obtained from a given time point up to present time, assuming that a period between (i) when a sign of a difference between the combined value obtained by the scalarizing means and the average calculated by the average calculating means changes from one to the opposite and (ii) when the sign changes from one to the opposite again is a step detection interval, the upper peak value and the lower peak value being a maximum value and a minimum value, respectively in the step detection interval.

**4.** Amended) The body movement measuring device as set forth in any one of claims 1 through 3, further comprising:

standard deviation calculating means for obtaining, at every given time, the combined value obtained by the scalarizing means, and calculating a standard deviation of the combined value obtained from a given time point up to present time; and average calculating means for obtaining, at every given time, the combined value obtained by the scalarizing means, and calculating an average of the combined value obtained from a given time point up to present time, the step counting means not counting another step when the requirement for counting of another step is met in any one of claims 1 through 3 but the upper peak value is not more than a sum of the average and the standard deviation or the lower peak value is not less than a difference between the average and the standard deviation.

**5.** Amended) The body movement measuring device as set forth in any one of claims 1 through 3, wherein the step counting means does not count another step when the requirement for counting of another step is met in any one of claims 1 through 3 but a period between (a) detection time of a lower peak value detected last time when another step was counted and (b) detection time of an upper peak value detected this time does not fall within a given range.

**6.** Amended) The body movement measuring device as set forth in any one of claims 1 through 3, further comprising:

average calculating means for obtaining, at every given time, the combined value obtained by the scalarizing means, and calculating an average of the combined value obtained from a given time point up to present time, the step counting means finding (i) a difference between the upper peak value and an average of the combined value obtained from the given time point to when the combined value reaches the upper peak value and (ii) a difference between the lower peak value and an average of the combined value obtained from the given time point to when the combined value reaches the lower peak value, wherein the step counting means does not count another step when the requirement for counting of another step is met in any one of claims 1 through 3 but a ratio between the differences (i) and (ii) does not fall within a given.

**7.** Amended) The body movement measuring device as set forth in any one of claims 1 through 3, further comprising:

a median calculating means for calculating a median of the upper peak value and the lower peak value which have been detected by the peak value detecting means, the step counting means finding (i) a difference between the upper peak value and the median and (ii) a difference between the lower peak value and the median, wherein the step counting means does not count another step when the requirement for counting of another step is met in any one of claims 1 through 3 but a ratio between the differences (i) and (ii) does not fall within a given range.

**8.** Amended) The body movement measuring device as set forth in any one of claims 1 through 7, wherein the step counting means does not count another step when a period between detection time of the upper peak value and detection time of the lower peak value does not fall within a given range.

**9.** Amended) The body movement measuring device as set forth in any one of claims 1 through 8, wherein:

the step counting means carries out determination of whether or not to count another step, for every one cycle of a waveform formed by the combined value; and

in a case where the step counting means does not count another step as a result of the determination, the step counting means carries out the determination by use of one of upper peak values and one of lower peak values in a period of a given number of cycles to a current cycle.

**10.** Amended) The body movement measuring device as set forth in claim 9, wherein the given number of cycles is one cycle before the current cycle.

**11.** Amended) The body movement measuring device as set forth in any one of claims 1 through 10, wherein:

the acceleration detecting section is an acceleration sensor for detecting accelerations in directions of three axes which cross each other orthogonally;

said body movement measuring device further includes quantizing means for quantizing outputs from the respective three axes of the acceleration sensor; and

the scalarizing means performs the scalarization for the three outputs quantized by the quantizing means.

**12.** Amended) The body movement measuring device as set forth in claim 11, wherein:

the acceleration sensor has a sensitivity which is a tenth of a power supply voltage per 1 G;

the quantizing means is an AD converter which has a quantization bit rate of 12; and

the given value used by the step counting means is 143.

**13.** Amended) The body movement measuring device as set forth in claim 6 or 7, wherein the given range recited in claim 6 or 7 ranges from one-fourth to four.

**14.** Amended) The body movement measuring device as set forth in claim 5, wherein the given range recited in claim 5 is not more than 100ms.

**15.** Amended) The body movement measuring device as set forth in claim 8, wherein the given range recited in claim 8 ranges from 100ms to 500ms.

**16.** Amended) The body movement measuring device as set forth in any one of claims 1 through 15, wherein the given time is 30ms.

**17.** Amended) A mobile phone comprising a body movement measuring device recited in any one of claims 1 through 16.

**18.** Amended) A body movement measuring device control program for causing a body movement measuring device recited in any one of claims 1 through 16 to operate, said body movement measuring device control program causing a computer to function as each means of the body movement measuring device.

**19.** New) A computer-readable recording medium in which the body movement measuring device control program recited in claim 18 is recorded.

**20.** New) A method for controlling a body movement measuring device including an acceleration detecting section for detecting accelerations in a plurality of directions,
said method comprising the steps of:

(a) combining the accelerations detected by the acceleration detecting section, so as to obtain a combined value, and then scalarizing the combined value thus obtained;

(b) sampling, at every given time, the combined value obtained by the scalarizing means, and detecting an upper peak value and a lower peak value of the combined value thus sampled; and

(c) counting another step when a difference between the upper peak value and the lower peak value which have been detected in the step (b) and are located adjacently on a time axis exceeds a given value.

**Statement under Art. 19.1 PCT**

Claim 2 is newly added. This is mainly supported by paragraph [0053] of the originally filed specification and Fig. 5.

Claim 3 is newly added. This is mainly supported by paragraphs [0045] through [0047] of the originally filed specification and Fig. 3.

Original claims 2 through 5 are renumbered to claims 4 through 7, respectively, so that claims 1 through 3 are clearly limited. This causes claims 1, 4, 6, and 7 to meet the requirements for unity.

Original claims 6 through 18 are renumbered to claims 8 through 20, respectively, and numbers of claims from which claims 8 through 20 depend are accordingly amended.

1

PORTABLE TERMINAL

10

CONTROL SECTION

11

X-AXIS DIRECTION BODY MOVEMENT SENSOR

12

Y-AXIS DIRECTION BODY MOVEMENT SENSOR

13

Z-AXIS DIRECTION BODY MOVEMENT SENSOR

101 QUANTIZING SECTION

102 OFFSET CORRECTION SECTION

103 SCALARIZING SECTION

104 MOVING AVERAGE CALCULATING SECTION

105 PEAK DETECTING SECTION

106 STEP COUNTING SECTION

COMMUNICATION SECTION

DISPLAY SECTION

INPUT SECTION

STORAGE SECTION

CLOCK SECTION

14

15

16

17

18

F I G. 2

```
        ┌──────────┐
        │  START   │
        └──────────┘
             │
   ┌─────────────────────┐
   │   Ax=ax−off_x       │──── S201
   └─────────────────────┘
             │
   ┌─────────────────────┐
   │   Ay=ay−off_y       │──── S202
   └─────────────────────┘
             │
   ┌─────────────────────┐
   │   Az=az−off_z       │──── S203
   └─────────────────────┘
             │
        ┌──────────┐
        │   END    │
        └──────────┘
```

F I G. 3

```
                    ( START )
                        │
                        │           ┌S301
              ┌─────────────────────┐
              │ RECEIVE INITIAL VALUE │
              └─────────────────────┘
                        │
                        │                    ┌S302
      ┌──────────────────────────────────────┐
      │ SET SIGN OF DIFFERENCE (A−MA) TO (+)  │
      └──────────────────────────────────────┘
                        │
                        │                    ┌S303
      ┌──────────────────────────────────────┐
      │ SET UP AND LP TO INITIAL VALUE  A0    │
      └──────────────────────────────────────┘
                        │
          ┌─────────────→
          │   ┌─────────→
          │   │          ┌S304
          │   │  ┌────────────────────────────┐
          │   │  │ RECEIVE SCALARIZING SECTION OUTPUT │
          │   │  │ SIGNAL (A) AND MOVING AVERAGE      │
          │   │  │ CALCULATING SECTION OUTPUT SIGNAL (MA) │
          │   │  └────────────────────────────┘
          │   │          ┌S305
          │   │  ┌──────────────────┐
          │   │  │ CALCULATE DIFFERENCE │
          │   │  │      (A−MA)          │
          │   │  └──────────────────┘
          │   │          ┌S306
          │   │       ◇ A>UP? ◇ ─── YES ───┐
          │   │          │                  │   ┌S307
          │   │          NO ←──────┌──────────────────┐
          │   │          │         │ DATA UPDATED TO   │
          │   │          │         │      UP = A       │
          │   │          │         └──────────────────┘
          │   │          │      ┌S308
          │   │       ◇ A<LP? ◇ ─── YES ───┐
          │   │          │                  │   ┌S309
          │   │          NO ←──────┌──────────────────┐
          │   │          │         │ DATA UPDATED TO   │
          │   │          │         │      LP = A       │
          │   │          │         └──────────────────┘
          │   │          │   ┌S310
          │   │       ◇ SIGN OF
          │   └── NO ── DIFFERENCE CHANGES
          │             FROM
          │             (−) TO (+)? ◇
          │                │
          │               YES
          │                │         ┌S311
          │       ┌──────────────────────────┐
          │       │   STEP DETECTION PROCESS  │
          │       └──────────────────────────┘
          │                │         ┌S312
          │       ┌──────────────────────────┐
          │       │ SET UP AND LP TO INITIAL VALUE A0 │
          │       └──────────────────────────┘
          │                │
          └────────────────┘
```

F I G. 4

```
              ┌─────────────────────┐
              │   STEP DETECTION     │
              │      PROCESS         │
              └─────────────────────┘
                        │
                        ▼
                    ╱ S401 ╲
                  ╱          ╲          NO
                ╱  UP—LP > α ? ╲ ─────────────────┐
                  ╲          ╱                     │
                    ╲      ╱                       │
                      YES                          │
                       │                           │
                       ▼                           │
                    ╱ S402 ╲                       │
                  ╱          ╲          NO          │
                ╱   equation   ╲ ───────────────→  │
                  ╲          ╱                      │
                    ╲      ╱                        │
                      YES                           │
                       │                            │
                       ▼                            │
                    ╱ S403 ╲                        │
                  ╱          ╲          NO           │
                ╱   equation   ╲ ──────────────→    │
                  ╲          ╱                       │
                    ╲      ╱                         │
                      YES                            │
                       │                             │
                       ▼                             │
              ┌─────────────────────┐  S404          │
              │  COUNT ANOTHER STEP │                │
              └─────────────────────┘                │
                       │◄────────────────────────────┘
                       ▼
                ┌─────────────┐
                │     END     │
                └─────────────┘
```

S401: $UP - LP > \alpha\,?$

S402: $\dfrac{1}{\beta} < \dfrac{UP - MA_{UP}}{MA_{LP} - LP} < \beta\,?$

S403: $\gamma < T_{LP} - T_{UP} < \delta\,?$

S404: COUNT ANOTHER STEP

29

FIG. 5

STEP DETECTION GRAPH 51

FIG. 6

PORTABLE TERMINAL 2

CONTROL SECTION 10

| 11 | | 101 | 102 | 103 | 104 | 105 | 107 | 61 |

X-AXIS DIRECTION BODY MOVEMENT SENSOR 11

Y-AXIS DIRECTION BODY MOVEMENT SENSOR 12

Z-AXIS DIRECTION BODY MOVEMENT SENSOR 13

QUANTIZING SECTION 101

OFFSET CORRECTION SECTION 102

SCALARIZING SECTION 103

MOVING AVERAGE CALCULATING SECTION 104

PEAK DETECTING SECTION 105

STEP COUNTING SECTION 107

RETAINED PEAK SETTING SECTION 61

COMMUNICATION SECTION 14

DISPLAY SECTION 15

INPUT SECTION 16

STORAGE SECTION 17

CLOCK SECTION 18

EP 2 260 763 A1

F I G. 7

START

S301
RECEIVE INITIAL VALUE

S302
SET SIGN OF DIFFERENCE
(A−MA) TO (+)

S303
SET UP AND LP TO
INITIAL VALUE $A_0$

S701
INVALIDATE PUP AND PLP

S304
RECEIVE SCALARIZING SECTION
OUTPUT SIGNAL (A) AND MOVING
AVERAGE CALCULATING SECTION
OUTPUT SIGNAL (MA)

S305
CALCULATE DIFFERENCE (A−MA)

S306
A>UP ?    YES

S307
DATA UPDATED TO
UP=A

NO

S308
A<LP ?    YES

S309
DATA UPDATED TO
LP=A

NO

S310
SIGN OF
DIFFERENCE CHANGES
FROM
(−) TO (+)?    NO

YES

S311
STEP DETECTION PROCESS

S702
CARRY OUT
STEP COUNTING?    YES

NO

S703
INVALIDATE
PUP AND PLP

S704
PUP AND PLP
VALID?    NO

YES

S705
STORE UP AND LP
AS PUP AND PLP

S706
UP>PUP ?    YES

NO

S707
DATA UPDATED TO
PUP = UP

S708
LP<PLP ?    YES

NO

S709
DATA UPDATED TO
PLP = LP

S710
RECEIPT TIME
OF PUP AND PLP FALLS
WITHIN GIVEN
RANGE?    NO

YES

S712
INVALIDATE
PUP AND PLP

S711
VALIDATE PUP AND PLP

S312
SET UP AND LP TO
INITIAL VALUE $A_0$

F I G. 8

```
                    ( START )
                        │
        ┌───────────────────────────────────┐ ╱ S801
        │  SUBJECT UP AND LP TO STEP DETECTION │
        └───────────────────────────────────┘
                        │
          ┌──┬─────────────────────┬──┐ ╱ S802
          │  │  STEP DETECTION      │  │
          │  │     PROCESS          │  │
          └──┴─────────────────────┴──┘
                        │
                      ◇ S803
        YES    ◇   DETECT STEP?   ◇
    ◄──────────◇                  ◇
                        │ NO
        ┌───────────────────────────────────┐ ╱ S804
        │   SUBJECT PUP AND PLP TO STEP      │
        │            DETECTION               │
        └───────────────────────────────────┘
                        │
          ┌──┬─────────────────────┬──┐ ╱ S805
          │  │  STEP DETECTION      │  │
          │  │     PROCESS          │  │
          └──┴─────────────────────┴──┘
                        │
                      ◇ S806
        YES    ◇   DETECT STEP?   ◇
    ◄──────────◇                  ◇
                        │ NO
        ┌───────────────────────────────────┐ ╱ S807
        │    SUBJECT UP AND LP TO STEP       │
        │            DETECTION               │
        └───────────────────────────────────┘
                        │
          ┌──┬─────────────────────┬──┐ ╱ S808
          │  │  STEP DETECTION      │  │
          │  │     PROCESS          │  │
          └──┴─────────────────────┴──┘
                        │
                    (  END  )
```

F I G. 9

STEP DETECTION GRAPH 91

F I G. 1 0

FALSE STEP COUNT

TRUE STEP COUNT

TIME

Legend:
- ● UPPER PEAK VALUE UP
- □ LOWER PEAK VALUE LP
- × COUNT DETERMINATION POINT P
- ◯ SURGING PART

F I G . 1 1

PORTABLE TERMINAL 3

CONTROL SECTION 10

11 X-AXIS DIRECTION BODY MOVEMENT SENSOR

12 Y-AXIS DIRECTION BODY MOVEMENT SENSOR

13 Z-AXIS DIRECTION BODY MOVEMENT SENSOR

101 QUANTIZING SECTION

102 OFFSET CORRECTION SECTION

103 SCALARIZING SECTION

104 MOVING AVERAGE CALCULATING SECTION

108 STANDARD DEVIATION CALCULATING SECTION

105 PEAK DETECTING SECTION

116 STEP COUNTING SECTION

14 COMMUNICATION SECTION

15 DISPLAY SECTION

16 INPUT SECTION

17 STORAGE SECTION

18 CLOCK SECTION

FIG.12

F I G. 1 3

```
      ┌─────────────────────┐
      │   STEP DETECTION     │
      │     PROCESS          │
      └─────────────────────┘
                 │
                 │           S1301
              ╱──┴──╲
            ╱         ╲              NO
          ╱  γ < T_LP - T_UP < δ ?  ╲──────────────┐
            ╲         ╱                             │
              ╲──┬──╱                               │
                 │ YES                              │
                 │           S1302                  │
              ╱──┴──╲                               │
            ╱         ╲        NO                   │
          ╱ UP-LP > ζ ? ╲──────────────────────────┤
            ╲         ╱                             │
              ╲──┬──╱                               │
                 │ YES                              │
      ┌─────────────────────┐                       │
      │ GIVE INSTURCTION ON │  S1303                │
      │  CALCULATION OF     │                       │
      │ STANDARD DEVIATION δ│                       │
      └─────────────────────┘                       │
                 │                                  │
      ┌─────────────────────┐  S1304                │
      │  OBTAIN STANDARD    │                       │
      │   DEVIATION δ       │                       │
      └─────────────────────┘                       │
                 │                                  │
                 │           S1305                  │
              ╱──┴──╲                               │
            ╱         ╲                             │
          ╱  LP < (MA-σ)  ╲      NO                 │
         ╱    AND          ╲────────────────────────┤
          ╲ (MA+σ) < UP?  ╱                         │
            ╲         ╱                             │
              ╲──┬──╱                               │
                 │ YES                              │
      ┌─────────────────────┐  S1306                │
      │ COUNT ANOTHER STEP  │                       │
      └─────────────────────┘                       │
                 │◄─────────────────────────────────┘
      ┌─────────────────────┐
      │        END          │
      └─────────────────────┘
```

FIG. 14

FIG. 15

F I G. 1 6

```
      ┌─────────────────────┐
      │   STEP DETECTION    │
      │      PROCESS        │
      └─────────────────────┘
               │
               │        S1601
            ╱─────────╲          NO
           ╱  UP—LP    ╲──────────────────┐
           ╲  > ζ ?    ╱                   │
            ╲─────────╱                    │
               │ YES                       │
               │                           │
               │              S1602        │
            ╱──────────────────╲    NO     │
           ╱   1    UP−MA_UP     ╲─────────┤
           ╲  ─── < ──────── < β ?╱        │
           ╱   β    MA_LP−LP     ╲         │
            ╲──────────────────╱           │
               │ YES                       │
               │              S1603        │
            ╱─────────────────╲     NO     │
           ╱  γ < T_LP − T_UP  ╲───────────┤
           ╲     < δ ?         ╱           │
            ╲─────────────────╱            │
               │ YES                       │
               │            S1604          │
            ╱───────────────╲       NO     │
           ╱  η < T_UP−T_WLP ╲─────────────┤
           ╲      ?          ╱             │
            ╲───────────────╱              │
               │ YES                       │
      ┌─────────────────────┐  S1605       │
      │ COUNT ANOTHER STEP  │              │
      └─────────────────────┘              │
               │                           │
               │◄──────────────────────────┘
      ┌─────────────────────┐
      │         END         │
      └─────────────────────┘
```

$$\frac{1}{\beta} < \frac{UP - MA_{UP}}{MA_{LP} - LP} < \beta \ ?$$

$$\gamma < T_{LP} - T_{UP} < \delta \ ?$$

$$\eta < T_{UP} - T_{WLP} \ ?$$

F I G. 1 7

```
        ┌─────────────────────┐
        │   STEP DETECTION    │
        │      PROCESS        │
        └─────────────────────┘
                  │
                  │      ┌S1701
                  ▼
             ╱─────────╲         NO
            ╱           ╲ ──────────────────►
            ╲ γ<T_LP−T_UP<δ ?   ╱
             ╲─────────╱
                  │ YES
                  │      ┌S1702
                  ▼
             ╱─────────╲         NO
            ╱           ╲ ──────────────────►
            ╲ η<T_UP−T_WLP ?    ╱
             ╲─────────╱
                  │ YES
                  │      ┌S1703
                  ▼
             ╱─────────╲         NO
            ╱  UP−LP>ζ ? ╲ ──────────────────►
            ╲           ╱
             ╲─────────╱
                  │ YES
                  ▼
        ┌─────────────────────┐
        │ GIVE INSTURCTION ON │  ┌S1704
        │   CALCULATION OF    │
        │ STANDARD DEVIATION σ │
        └─────────────────────┘
                  │
                  ▼
        ┌─────────────────────┐
        │  OBTAIN STANDARD    │  ┌S1705
        │    DEVIATION σ      │
        └─────────────────────┘
                  │
                  │      ┌S1706
                  ▼
             ╱─────────╲
            ╱ LP<(MA−σ) ╲        NO
            ╲    AND    ╱ ──────────────────►
            ╲(MA+σ)<UP? ╱
             ╲─────────╱
                  │ YES
                  ▼
        ┌─────────────────────┐  ┌S1707
        │  COUNT ANOTHER STEP │
        └─────────────────────┘
                  │
                  ▼
        ┌─────────────────────┐
        │         END         │
        └─────────────────────┘
```

Conditions as written:

- S1701: $\gamma < T_{LP} - T_{UP} < \delta$ ?
- S1702: $\eta < T_{UP} - T_{WLP}$ ?
- S1703: $UP - LP > \zeta$ ?
- S1704: GIVE INSTURCTION ON CALCULATION OF STANDARD DEVIATION $\sigma$
- S1705: OBTAIN STANDARD DEVIATION $\sigma$
- S1706: $LP < (MA - \sigma)$ AND $(MA + \sigma) < UP$ ?
- S1707: COUNT ANOTHER STEP

F I G. 1 8

F I G. 1 9

F I G. 2 0

```
        ┌──────────────────────┐
        │   STEP DETECTION     │
        │      PROCESS         │
        └──────────────────────┘
                   │
                   │            S2001
              ◇─────────────◇
         ╱                       ╲        NO
        ◇  γ < T_{LP} − T_{UP} < δ ?  ◇─────────→
         ╲                       ╱
              ◇─────────────◇
                   │ YES
                   │            S2002
              ◇─────────────◇
    YES  ╱                   ╲
    ←───◇    UP−LP > α ?      ◇
         ╲                   ╱
              ◇─────────────◇
                   │ NO
                   │            S2003
              ◇─────────────◇
         ╱                   ╲        NO
        ◇    UP−LP > ζ ?      ◇─────────────→
         ╲                   ╱
              ◇─────────────◇
                   │ YES
        ┌──────────────────────┐
        │  GIVE INSTURCTION ON │   S2004
        │   CALCULATION OF     │
        │ STANDARD DEVIATION σ │
        └──────────────────────┘
                   │
        ┌──────────────────────┐
        │  OBTAIN STANDARD     │   S2005
        │   DEVIATION σ        │
        └──────────────────────┘
                   │            S2006
              ◇─────────────◇
         ╱                   ╲
        ◇    LP < (MA−σ)      ◇        NO
        ◇       AND           ◇─────────────→
        ◇  (MA+σ) < UP?       ◇
         ╲                   ╱
              ◇─────────────◇
                   │ YES
        ┌──────────────────────┐
        │  COUNT ANOTHER STEP  │   S2007
        └──────────────────────┘
                   │
        ┌──────────────────────┐
        │        END           │
        └──────────────────────┘
```

F I G. 2 1

$$\text{STEP DETECTION PROCESS}$$

S2101

$\gamma < T_{LP} - T_{UP} < \delta$ ? — NO

YES

S2102

$\eta < T_{UP} - T_{WLP}$ ? — NO

YES

S2103

UP−LP > $\alpha$ ? — YES

NO

S2104

UP−LP > $\zeta$ ? — NO

YES

GIVE INSTURCTION ON CALCULATION OF STANDARD DEVIATION $\sigma$ — S2105

OBTAIN STANDARD DEVIATION $\sigma$ — S2106

S2107

$LP < (MA - \sigma)$ AND $(MA + \sigma) < UP$? — NO

YES

COUNT ANOTHER STEP — S2108

END

## INTERNATIONAL SEARCH REPORT

| | International application No. |
|---|---|
| | PCT/JP2009/052579 |

| A. CLASSIFICATION OF SUBJECT MATTER |
|---|
| *A61B5/11*(2006.01)i, *G01C22/00*(2006.01)i, *G06M3/00*(2006.01)i |

According to International Patent Classification (IPC) or to both national classification and IPC

| B. FIELDS SEARCHED |
|---|
| Minimum documentation searched (classification system followed by classification symbols) |
| A61B5/11, G01C22/00, G06M3/00 |

| Documentation searched other than minimum documentation to the extent that such documents are included in the fields searched |
|---|
| Jitsuyo Shinan Koho 1922-1996 Jitsuyo Shinan Toroku Koho 1996-2009 |
| Kokai Jitsuyo Shinan Koho 1971-2009 Toroku Jitsuyo Shinan Koho 1994-2009 |

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| A | JP 2005-38018 A (Asahi Kasei EMD Corp.), 10 February, 2005 (10.02.05), Claims; Par. Nos. [0014] to [0021], [0029] (Family: none) | 1-18 |
| A | JP 2008-54768 A (Aisin Seiki Co., Ltd.), 13 March, 2008 (13.03.08), Claim 1 (Family: none) | 1-18 |
| A | JP 2007-148702 A (Matsushita Electric Works, Ltd.), 14 June, 2007 (14.06.07), Abstract; Par. Nos. [0036] to [0037] (Family: none) | 1-18 |

☒ Further documents are listed in the continuation of Box C. ☐ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 01 May, 2009 (01.05.09) | 19 May, 2009 (19.05.09) |

| Name and mailing address of the ISA/ | Authorized officer |
|---|---|
| Japanese Patent Office | |
| Facsimile No. | Telephone No. |

Form PCT/ISA/210 (second sheet) (April 2007)

EP 2 260 763 A1

**INTERNATIONAL SEARCH REPORT**

| | International application No. |
|---|---|
| | PCT/JP2009/052579 |

C (Continuation). DOCUMENTS CONSIDERED TO BE RELEVANT

| Category* | Citation of document, with indication, where appropriate, of the relevant passages | Relevant to claim No. |
|---|---|---|
| A | JP 2006-180899 A (Sharp Corp.), 13 July, 2006 (13.07.06), Par. Nos. [0021] to [0023], [0043] to [0045], [0055]; Fig. 1 (Family: none) | 15 |

Form PCT/ISA/210 (continuation of second sheet) (April 2007)

48

## INTERNATIONAL SEARCH REPORT

International application No.

PCT/JP2009/052579

| Box No. II | Observations where certain claims were found unsearchable (Continuation of item 2 of first sheet) |
|---|---|

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

| Box No. III | Observations where unity of invention is lacking (Continuation of item 3 of first sheet) |
|---|---|

This International Searching Authority found multiple inventions in this international application, as follows:
   The search has revealed that the matter common to the individual inventions of claims 1, 2, 4 and 5 is not novel, since it was disclosed in JP 2005-38018 A (Asahi Kasei EMD Corp.), 10 February, 2005 (10.02.05), [Claims]. Par. Nos. [0014] - [0021] and [0029]. As a result, the matter common to claims 1, 2, 4 and 5 is not the special technical feature within the meaning of PCT Rule 13.2, second sentence, since it makes no contribution over the prior art. Hence, there exists no matter common to all the inventions of claims 1, 2, 4 and 5. It is admitted that this application contains the following invention groups which do not comply with the requirement of unity of invention.
   (continued to extra sheet)

1. ☒ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**
the

☐ The additional search fees were accompanied by the applicant's protest and, where applicable, payment of a protest fee.

☐ The additional search fees were accompanied by the applicant's protest but the applicable protest fee was not paid within the time limit specified in the invitation.

☒ No protest accompanied the payment of additional search fees.

Form PCT/ISA/210 (continuation of first sheet (2)) (April 2007)

**INTERNATIONAL SEARCH REPORT**

| International application No. |
| --- |
| PCT/JP2009/052579 |

<u>Continuation of Box No.III of continuation of first sheet(2)</u>

Invention group 1: claims 1, 6 - 17 (if they accord to claim 1) and 18
Invention group 2: claims 2, 3 and 6 - 17 (if they accord to claim 2 or 3)
Invention group 3: claims 4 and 6 - 17 (if they accord to claim 4)
Invention group 4: claims 5 and 6 - 17 (if they accord to claim 5)

Form PCT/ISA/210 (extra sheet) (April 2007)

**REFERENCES CITED IN THE DESCRIPTION**

**Patent documents cited in the description**

- JP 2006122573 A

- JP 2001143048 A